# EUROPEAN PATENT APPLICATION

(11) **EP 4 470 535 A1**
(43) Date of publication of application: **04.12.2024**
(21) Application number: 23747315.2
(22) Date of filing: 25.01.2023
(51) Int. Cl.: A61K 31/36, A61K 31/506, A61K 45/06, A61P 17/14, A61P 17/00, A61K 8/49, A61Q 7/00, A61Q 5/00, A23L 33/10

(54) **COMPOSITION FOR PREVENTING, ALLEVIATING OR TREATING ALOPECIA OR POLIOSIS THROUGH ALDEHYDE DEHYDROGENASE 2 REGULATION**

(30) Priority: 26.01.2022 KR 20220011538; 29.06.2022 KR 20220079800; 15.11.2022 KR 20220152981
(71) Applicant: Seoul National University Hospital, Seoul 03080 (KR)
(72) Inventor: KWON, Ohsang, Seoul 06519 (KR); LEE, Seunghee, Seoul 04105 (KR); OHN, Jungyoon, Seoul 03130 (KR)
(74) Representative: Dehns
(86) International application number: PCT/KR2023/001139
(87) International publication number: WO 2023/146274

(57) **Abstract**

The present invention relates to a composition for preventing, alleviating and/or treating alopecia or gray hair (or poliosis), and the like, and was completed by confirming that an aldehyde dehydrogenase 2 (ALDH2) activator promotes hair follicle formation and hair growth and has an excellent effect on inhibiting the oxidative stress of hair follicles. Therefore, the ALDH2 activator according to the present invention can be used as a preparation for preventing, alleviating and/or treating alopecia and gray hair. Furthermore, combined treatment of the ALDH2 activator and minoxidil exhibited better alopecia prevention and treatment effects. Therefore, ALDH2 according to the present invention not only effectively promotes hair growth by itself, but also is expected to be usefully used in combination with minoxidil in the medical, beauty and food fields in order to prevent, alleviate and treat alopecia.

## Description

### [Technical Field]

The present invention relates to a composition for preventing, alleviating or treating alopecia or gray hair, and the like by regulating aldehyde dehydrogenase 2. Further, the present invention relates to a combination therapy for preventing, alleviating or treating alopecia by regulating aldehyde dehydrogenase 2.

The present invention claims priority to and the benefit of Korean Patent Application Nos. 10-2022-0011538, 10-2022-0079800, and 10-2022-0152981 filed in the Korean Intellectual Property Office on January 26, 2022, June 29, 2022, and November 15, 2022, respectively, and all the contents disclosed in the specification and drawings of these applications are incorporated in the present application.

### [Background Art]

Hair follicles, which are organs that produce hair, repeat a hair cycle that is divided into the anagen phase, in which hair is produced and the produced hair actively grows, the catagen phase, in which the hair degenerates, the telogen phase, in which the hair is maintained until it falls out, and the exogen phase, in which hair loss occurs, and are involved in the growth, maintenance, and shedding of hair. It is known that during the anagen phase of hair follicles, in adults, hair grows at an average rate of about 0.3 mm per day and about 1 cm per month, and continues to grow for about 5 to 8 years. Generally, hair falls out after the anagen phase, the catagen phase lasting about three weeks, and the telogen phase lasting an average of three months. The reason why human hair length varies from area to area is that the duration of the anagen phase, which is an inherent characteristic of hair follicles, differs from area to area.

Hair is formed in hair follicles. The hair follicle is a very complex organ, is composed of the inner root sheath, outer root sheath, hair shaft, hair matrix cells, and the like, and in particular, hair dermal papilla cells play a key role in hair follicle formation, and thus are directly linked to the ability to form hair follicles. When hair dermal papilla cells are absent or do not function, hair follicles cannot be formed because appropriate interactions between hair dermal papilla cells and epidermal cells do not occur.

Alopecia refers to a state in which there is little or no hair in areas where hair should normally be present. It is known that factors such as genetic causes or the effects of male hormones, and the like, endocrine disorders, nutritional deficiencies, drug use, and severe physical and mental stress such as childbirth, fever, and surgery are involved in alopecia in a complex manner. Recently, not only male pattern alopecia but also the number of women experiencing alopecia has been increasing due to changes in diet and increased stress caused by the social environment, and the like, and as a result, the number of people suffering from abnormal symptoms of the scalp and hair is increasing and the age of those suffering from the abnormal symptoms is also becoming younger.

Although research into alopecia prevention and hair regrowth agents is accelerating both at home and abroad and various products are being developed, there is still a lack of development of alopecia prevention agents that have excellent efficacy and are safe for the human body. Currently developed topical products include minoxidil in the USA, and herbal extracts are being developed primarily in Japan as hair growth or hair regrowth agents, but it is still too early to make claims about alopecia prevention and hair regrowth functions. In particular, minoxidil has been reported to cause side effects such as body weight gain, edema, increased heart rate, angina pectoris, dermatitis, and itching when used in high doses. Therefore, there is a need for discovering a more effective alopecia therapeutic agent capable of replacing minoxidil or a combination therapy capable of enhancing the hair growth effect of minoxidil and reducing the side effects.

Currently, 5α-reductase inhibitors finasteride and dutasteride are almost the only effective treatments for male pattern baldness, but treatment effectiveness varies greatly from person to person, and alopecia may recur or serious side effects such as sexual dysfunction may occur when the use of the product is discontinued. In particular, since most of the existing alopecia therapeutic agents approved by the US FDA are drugs developed with a focus on male pattern alopecia, they have limitations in addressing female pattern alopecia and are rather likely to cause side effects in women. Therefore, there is a great need for research into new alopecia prevention and hair regrowth agents capable of being proven to be safe and effective for both men and women.

Gray hair is one of the aging processes and occurs in various ways depending on the individual. Although the cause of gray hair is understood to be the loss of or decreased activity of melanocytes in the hair bulb and outer root sheath, the mechanism in cells or molecules is not clearly known. Amelanotic melanocytes remain in the outer root sheath of gray hair, but are not found in the hair bulb, suggesting a reduction in melanocyte stem cells or an abnormality in the processes regulating the migration and differentiation of melanocytes. Melanocytes, located in the hair bulb of human hair follicles, begin to be organized around the dermal papilla during the anagen phase of hair after division. Tyrosinase and TRP-1 enzymes expressed in melanocytes are only observed during the anagen phase and are not produced during the catagen phase. Therefore, pigment may be regenerated in the hair at the top of the outer root sheath through melanocytes that are periodically activated during the anagen phase.

The accumulation of reactive oxygen species generated during the melanin production process damages the antioxidant system to induce oxidative stress. Excessive oxidative stress is known to affect the production of melanocytes. In particular, accumulated oxidative stress may disrupt the homeostasis of melanocytes to induce the death or deformation of cells. Therefore, in order to prevent or alleviate not only alopecia but also gray hair, it is extremely important to suppress oxidative stress in melanocytes.

Since gray hair gives a strong impression of old age, much research has been conducted into dyes which dye gray hair black in order to hide the gray hair that has been produced. However, dyeing is only a temporary method and since it is based on chemicals, it can damage the scalp and worsen the health of hair follicles. Therefore, there is a need for discovering a new method capable of fundamentally preventing and alleviating gray hair.

Meanwhile, it is known that aldehyde dehydrogenase 2 (ALDH2) is an enzyme present in mitochondria in the body, and usually converts acetaldehyde into acetic acid to help break down alcohol absorbed in the body. However, the effect of activating the aforementioned enzyme on preventing alopecia or promoting hair regrowth has not yet been reported.

### [Disclosure]

### [Technical Problem]

The present invention has been devised to solve the above problems, and has been completed by confirming that an aldehyde dehydrogenase 2 activator promotes hair follicle formation and hair growth, and is thus effective for preventing alopecia, promoting hair regrowth, and preventing or alleviating gray hair. Furthermore, it has been confirmed that an aldehyde dehydrogenase 2 activator may exhibit a synergistic alopecia treatment effect when used in combination with minoxidil.

The present invention is directed to providing a pharmaceutical composition for preventing or treating alopecia or gray hair, including an aldehyde dehydrogenase 2 activator as an active ingredient.

The present invention is directed to providing a cosmetic composition for preventing or alleviating alopecia or gray hair, including an aldehyde dehydrogenase 2 activator as an active ingredient.

The present invention is also directed to providing a quasi-drug composition for preventing or alleviating alopecia or gray hair, including an aldehyde dehydrogenase 2 activator as an active ingredient.

The present invention is also directed to providing a food composition for preventing or alleviating alopecia or gray hair, including an aldehyde dehydrogenase 2 activator as an active ingredient. The food composition includes a health functional food composition.

The present invention is also directed to providing a pharmaceutical composition for preventing or treating alopecia, including an aldehyde dehydrogenase 2 activator as an active ingredient, wherein the pharmaceutical composition is administered in combination with minoxidil, a pharmaceutically acceptable salt thereof, or an analog thereof.

The present invention is also directed to providing a pharmaceutical composition for preventing or treating alopecia, including (a) an aldehyde dehydrogenase 2 activator; and (b) minoxidil, a pharmaceutically acceptable salt thereof, or an analog thereof, as active ingredients.

The present invention is also directed to providing a cosmetic composition for preventing or alleviating alopecia, including (a) an aldehyde dehydrogenase 2 activator; and (b) minoxidil, a cosmetically acceptable salt thereof, or an analog thereof, as active ingredients.

The present invention is also directed to providing a quasi-drug composition for preventing or alleviating alopecia, including (a) an aldehyde dehydrogenase 2 activator; and (b) minoxidil, a pharmaceutically acceptable salt thereof, or an analog thereof, as active ingredients.

The present invention is also directed to providing a food composition for preventing or alleviating alopecia, including (a) an aldehyde dehydrogenase 2 activator; and (b) minoxidil, a sitologically acceptable salt thereof, or an analog thereof, as active ingredients.

The present invention is also directed to providing a health functional food composition for preventing or alleviating alopecia, including (a) an aldehyde dehydrogenase 2 activator; and (b) minoxidil, a sitologically acceptable salt thereof, or an analog thereof, as active ingredients.

However, the technical problems which the present invention intends to solve are not limited to the technical problems that have been mentioned above, and other technical problems that have not been mentioned will be clearly understood by a person with ordinary skill in the art to which the present invention pertains from the following description.

### [Technical Solution]

One aspect of the present invention provides a pharmaceutical composition for preventing or treating alopecia or gray hair, including an aldehyde dehydrogenase 2 (ALDH2) activator as an active ingredient.

Another aspect of the present invention provides a method for preventing or treating alopecia or gray hair, the method including administering an ALDH2 activator to a subject in need thereof.

Still another aspect of the present invention provides a use of an ALDH2 activator for preventing or treating alopecia or gray hair.

Yet another aspect of the present invention provides a use of an ALDH2 activator for preparing a drug for treating alopecia or gray hair.

In an exemplary embodiment of the present invention, the composition may be for promoting hair growth or hair regrowth, but is not limited thereto.

In another exemplary embodiment of the present invention, the alopecia may be any one or more selected from the group consisting of alopecia areata, hereditary androgenetic alopecia, telogen effluvium, traumatic alopecia, trichotillomania, pressure alopecia, anagen alopecia, pityriasis alopecia, syphilitic alopecia, seborrheic alopecia, symptomatic alopecia, and congenital alopecia, but is not limited thereto.

In still another exemplary embodiment of the present invention, the aldehyde dehydrogenase 2 activator may be Alda-1 or a pharmaceutically acceptable salt thereof, but is not limited thereto.

In yet another exemplary embodiment of the present invention, the Alda-1 or the pharmaceutically acceptable salt thereof may be contained at a concentration of 0.0001 to 50 mM with respect to the composition, but is not limited thereto.

In yet another exemplary embodiment of the present invention, the composition may satisfy one or more characteristics selected from the group consisting of, but not limited to: (a) promoting the proliferation of hair dermal papilla cells or outer root sheath cells; (b) promoting the growth of hair follicles; (c) inducing the anagen phase of hair; and (d) increasing mitochondrial activity.

In yet another exemplary embodiment of the present invention, the composition may increase the level or activity of one or more proteins selected from the group consisting of, but not limited to, bFGF, FGF-7, IGF-1, and PDGF.

In yet another exemplary embodiment of the present invention, the composition may reduce oxidative stress in hair dermal papilla cells or outer root sheath cells, but is not limited thereto.

Yet another aspect of the present invention provides a cosmetic composition for preventing or alleviating alopecia or gray hair, including an ALDH2 activator as an active ingredient.

In an exemplary embodiment of the present invention, the composition may be a formulation for external use in the form of a scalp treatment agent, soap, hair tonic, shampoo, rinse, hair pack, hair gel, lotion, conditioner, hair oil, mousse, cream, a solid agent, a solution, an emulsion, a dispersant, a micelle, a liposome, an ointment, toilet water, an essence, a patch, or a spray, but is not limited thereto.

Yet another aspect of the present invention provides a quasi-drug composition for preventing or alleviating alopecia or gray hair, including an ALDH2 activator as an active ingredient.

Yet another aspect of the present invention provides a food composition for preventing or alleviating alopecia or gray hair, including an ALDH2 activator as an active ingredient.

Yet another aspect of the present invention provides a health functional food composition for preventing or alleviating alopecia or gray hair, including the food composition as an active ingredient.

Yet another aspect of the present invention provides a method for preventing or alleviating gray hair, the method including administering an ALDH2 activator to a subject in need thereof. The method may be a cosmetic method.

Yet another aspect of the present invention provides a use of an ALDH2 activator for preventing or alleviating gray hair.

Yet another aspect of the present invention provides a use of an ALDH2 activator for preparing a drug for preventing or alleviating gray hair.

Yet another aspect of the present invention provides a pharmaceutical composition for preventing or treating alopecia, including an aldehyde dehydrogenase 2 activator as an active ingredient, which is administered in combination with minoxidil, a pharmaceutically acceptable salt thereof, or an analog thereof.

Yet another aspect of the present invention provides a use of an aldehyde dehydrogenase 2 activator administered in combination with minoxidil, a pharmaceutically acceptable salt thereof, or an analog thereof.

Yet another aspect of the present invention provides a method for preventing or treating alopecia, the method including administering, to a subject in need thereof, (a) an aldehyde dehydrogenase 2 activator; and (b) minoxidil, a pharmaceutically acceptable salt thereof, or an analog thereof.

Yet another aspect of the present invention provides a use of a composition including: (a) an aldehyde dehydrogenase 2 activator; and (b) minoxidil, a pharmaceutically acceptable salt thereof, or an analog thereof, for preventing or treating alopecia.

Yet another aspect of the present invention provides a use of a composition including: (a) an aldehyde dehydrogenase 2 activator; and (b) minoxidil, a pharmaceutically acceptable salt thereof, or an analog thereof, for preparing a drug for preventing or treating alopecia.

In an exemplary embodiment of the present invention, the pharmaceutical composition may be administered simultaneously or sequentially with minoxidil, a pharmaceutically acceptable salt thereof, or an analog thereof, but is not limited thereto.

In another exemplary embodiment of the present invention, the composition may be for promoting hair growth or hair regrowth, but is not limited thereto.

In still another exemplary embodiment of the present invention, the aldehyde dehydrogenase 2 activator may enhance the alopecia treatment effect of minoxidil, a pharmaceutically acceptable salt thereof, or an analog thereof, but is not limited thereto.

In yet another exemplary embodiment of the present invention, the aldehyde dehydrogenase 2 activator may be Alda-1 or a pharmaceutically acceptable salt thereof, but is not limited thereto.

In yet another exemplary embodiment of the present invention, the Alda-1 or the pharmaceutically acceptable salt thereof may be included at a concentration of 0.0001 to 50 mM with respect to the total composition, but is not limited thereto.

In yet another exemplary embodiment of the present invention, the composition may satisfy one or more characteristics selected from the group consisting of, but not limited to: (a) promoting the proliferation of hair dermal papilla cells or outer root sheath cells; (b) promoting the growth of hair follicles; (c) inducing the anagen phase of hair; and (d) increasing mitochondrial activity.

In yet another exemplary embodiment of the present invention, the composition may increase the level or activity of one or more proteins selected from the group consisting of, but not limited to, bFGF, FGF-7, IGF-1, and PDGF.

In yet another exemplary embodiment of the present invention, the composition may reduce oxidative stress in hair dermal papilla cells or outer root sheath cells, but is not limited thereto.

Yet another aspect of the present invention provides a pharmaceutical composition for preventing or treating alopecia, including (a) an aldehyde dehydrogenase 2 activator; and (b) minoxidil, a pharmaceutically acceptable salt thereof, or an analog thereof, as active ingredients.

In an exemplary embodiment of the present invention, the aldehyde dehydrogenase 2 activator may be Alda-1 or a pharmaceutically acceptable salt thereof, but is not limited thereto.

In another exemplary embodiment of the present invention, the composition may be in the form of a mixed preparation in which the (a) aldehyde dehydrogenase 2 activator; and the (b) minoxidil, a pharmaceutically acceptable salt thereof, or an analog thereof are mixed, but is not limited thereto.

In still another exemplary embodiment of the present invention, the composition may be in a form in which the (a) aldehyde dehydrogenase 2 activator; and the (b) minoxidil, a pharmaceutically acceptable salt thereof, or an analog thereof are each formulated and administered simultaneously, separately, or sequentially, but is not limited thereto.

Yet another aspect of the present invention provides a cosmetic composition for preventing or alleviating alopecia, including (a) an aldehyde dehydrogenase 2 activator; and (b) minoxidil, a pharmaceutically (or cosmetically) acceptable salt thereof, or an analog thereof, as active ingredients.

In an exemplary embodiment of the present invention, the composition may be a formulation for external use in the form of a scalp treatment agent, soap, hair tonic, shampoo, rinse, hair pack, hair gel, lotion, conditioner, hair oil, mousse, cream, a solid agent, a solution, an emulsion, a dispersant, a micelle, a liposome, an ointment, toilet water, an essence, a patch, or a spray, but is not limited thereto.

Yet another aspect of the present invention provides a quasi-drug composition for preventing or alleviating alopecia, including (a) an aldehyde dehydrogenase 2 activator; and (b) minoxidil, a pharmaceutically acceptable salt thereof, or an analog thereof, as active ingredients.

Yet another aspect of the present invention provides a cosmetic composition for preventing or alleviating alopecia, including (a) an aldehyde dehydrogenase 2 activator; and (b) minoxidil, a pharmaceutically (or sitologically) acceptable salt thereof, or an analog thereof, as active ingredients.

Yet another aspect of the present invention provides a health functional food composition for preventing or alleviating alopecia, including (a) an aldehyde dehydrogenase 2 activator; and (b) minoxidil, a pharmaceutically (or sitologically) acceptable salt thereof, or an analog thereof, as active ingredients.

Yet another aspect of the present invention provides a method for preventing or alleviating alopecia, the method including administering a composition including: (a) an aldehyde dehydrogenase 2 activator; and (b) minoxidil, a pharmaceutically acceptable salt thereof, or an analog thereof to a subject in need thereof. The method may be a cosmetic method.

Yet another aspect of the present invention provides a use of a composition including: (a) an aldehyde dehydrogenase 2 activator; and (b) minoxidil, a pharmaceutically acceptable salt thereof, or an analog thereof, for preventing or alleviating alopecia.

Yet another aspect of the present invention provides a use of a composition including: (a) an aldehyde dehydrogenase 2 activator; and (b) minoxidil, a pharmaceutically acceptable salt thereof, or an analog thereof, for preparing a drug for preventing or alleviating alopecia.

Yet another aspect of the present invention provides a pharmaceutical composition including an aldehyde dehydrogenase 2 activator as an active ingredient, for use in combination with an alopecia therapeutic agent.

Yet another aspect of the present invention provides a use of an aldehyde dehydrogenase 2 activator for preparing a combination preparation with an alopecia therapeutic agent.

### [Advantageous Effects]

The present invention relates to a composition for preventing, alleviating and/or treating alopecia or gray hair, and the like, and was completed by confirming that an aldehyde dehydrogenase 2 (ALDH2) activator promotes hair follicle formation and hair growth and has an excellent effect in inhibiting the oxidative stress of hair follicles. Specifically, the present inventors have confirmed that the ALDH2 activator promotes the proliferation of hair dermal papilla cells and outer root sheath cells, which are key cells in hair follicle formation and hair growth, and promotes cell division in hair follicles, and have confirmed that the anagen phase of hair growth is induced when a mouse model is treated with the ALDH2 activator. Further, it has been confirmed that treatment with the ALDH2 activator increases the levels of hair growth-promoting factors in hair dermal papilla cells, and the like, and inhibits oxidative stress, which is the cause of alopecia and gray hair. As described above, the ALDH2 activator according to the present invention can effectively promote the formation and growth of hair follicles, and thus can be used as a preparation for preventing, alleviating, and/or treating alopecia and gray hair. In particular, when a mouse model is treated with the ALDH2 activator according to the present invention, the activator exhibits the same or better effect than minoxidil which is an alopecia therapeutic agent, even at a much smaller dose, so it is expected not only to have an effect of reducing costs in the development of a therapeutic agent, but also to make the therapeutic agent safer for clinical application.

Furthermore, combination treatment of the ALDH2 activator and minoxidil exhibited better alopecia prevention and treatment effects. Therefore, since ALDH2, itself, according to the present invention, not only effectively promotes hair growth but also achieves a synergistic alopecia treatment effect when used in combination with minoxidil, it is expected to be usefully used in the medical, beauty and food fields for the purpose of preventing, alleviating and treating alopecia.

### [Description of Drawings]

FIG. 1A shows the results of treating hair dermal papilla cells derived from human hair follicles with an ALDH2 activator (Alda-1, hereinafter the same) according to an exemplary embodiment of the present invention and performing a CCK8 analysis to confirm the effect of the ALDH2 activator on the proliferation of hair dermal papilla cells.
FIG. 1B shows the results of treating outer root sheath cells derived from human hair follicles with an ALDH2 activator and performing a CCK8 analysis to confirm the effect of ALDH2 activation on the proliferation of outer root sheath cells.
FIGS. 2A and 2B are views showing the results of performing a human hair follicle organ culture test in order to confirm the effect of ALDH2 activation on hair growth in hair follicles. FIG. 2A shows the hair elongation effect in hair follicles treated with an ALDH2 activator, and FIG. 2B shows the results of performing immunofluorescence staining with a Ki-67 antibody in order to confirm the proliferation of cells in hair follicles treated with the ALDH2 activator and cultured.
FIG. 3 exhibits the results of treating a mouse model with an ALDH2 activator and comparing the extent to which hair during the anagen phase was grown in each group in order to confirm the effect of ALDH2 activation on inducing the anagen phase.
FIG. 4 shows the results of histological analysis of the degree of hair follicle formation during the anagen phase of a mouse model according to ALDH2 activation through hematoxylin and eosin staining.
FIG. 5A shows the results of confirming the anagen phase induction score by the ALDH2 activator in a mouse model.
FIG. 5B shows the results of confirming changes in skin thickness caused by the ALDH2 activator in a mouse model.
FIG. 6 shows the results of confirming the protein levels of hair growth-promoting factors by an ELISA assay method after treating hair dermal papilla cells and outer root sheath cells derived from human hair follicles with an ALDH2 activator in order to confirm the hair growth-promoting mechanism of the ALDH2 activator.
FIG. 7 shows the results of detecting fluorescently labeled DCFDA after treating hair dermal papilla cells with an ALDH2 activator to confirm the oxidative stress inhibitory effect of the ALDH2 activator.
FIGS. 8A and 8B show the results of measuring a DCFDA level (FIG. 8A) and the results of confirming a ROS scavenging ratio (FIG. 8B) after treating outer root sheath cells with an ALDH2 activator, in order to confirm the oxidative stress inhibitory effect of the ALDH2 activator.
FIG. 9A shows a set of images taken after fluorescently staining a mitochondrial outer membrane protein (VDAC, green) and an ALDH2 protein (red) in alopecia patient tissue (blue: DPAI).
FIG. 9B shows the results of comparing the expression levels of ALDH2 in hair follicles during the telogen phase and in hair follicles during the anagen phase.
FIG. 10A shows the results of confirming the oxygen consumption efficiency of outer root sheath cells derived from human hair follicles after treating the cells with an ALDH2 activator in order to confirm changes in mitochondrial activity according to the activity of ALDH2 in human hair follicles.
FIGS. 10B to 10D show the results of measuring the basal respiration rate (FIG. 10B), maximal respiration rate (FIG. 10C), and mitochondrial ATP production (FIG. 10D) of outer root sheath cells derived from human hair follicles after treatment with an ALDH2 activator.
FIG. 11 shows the results of observing the degree of hair growth (top) and the results of detecting a hair dermal papilla cell marker versican by immunofluorescence staining (bottom) after applying an ALDH2 activator and/or minoxidil to the backs of shaved mice in order to confirm the effect of the combined use of the ALDH2 activator and minoxidil on the treatment of alopecia.

### [Modes of the Invention]

The present invention relates to a composition for preventing, alleviating and/or treating alopecia or gray hair (or poliosis), and the like, and was completed by confirming that an aldehyde dehydrogenase 2 (ALDH2) activator promotes hair follicle formation and hair growth and has an excellent effect in inhibiting the oxidative stress of hair follicles.

Further, the present invention relates to a combination therapy for preventing, alleviating, and/or treating alopecia, and was completed by confirming that an aldehyde dehydrogenase 2 (ALDH2) activator not only promotes hair follicle formation and growth and inhibits oxidative stress in hair follicles, thereby exerting not only excellent alopecia treatment effects by itself, but also even more remarkable alopecia treatment effects when used in combination with minoxidil.

Specifically, in an example of the present invention, it was confirmed that the proliferation of hair dermal papilla cells and outer root sheath cells derived from human hair follicles was promoted during treatment with an ALDH2 activator Alda-1 (Example 1).

In another example of the present invention, as a result of treating hair follicles with Alda-1 and culturing the hair follicles through a human hair follicle organ culture test, it was confirmed that hair elongation and cell proliferation were increased in the hair follicles (Example 2).

In still another example of the present invention, as a result of applying Alda-1 to a hair-removed mouse model, and then comparing the degrees of hair growth and performing a histological analysis, it was confirmed that the anagen phase of hair was induced by ALDH2 activation to increase hair growth and also increase skin thickness (Example 3).

In yet another example of the present invention, as a result of confirming the expression levels of hair growth-promoting factors by treating hair dermal papilla cells with Alda-1, it was confirmed that the protein levels of bFGF, FGF-7, IGF-1, and PDGF were increased by ALDH2 activation (Example 4).

In yet another example of the present invention, it was confirmed that oxidative stress in hair dermal papilla cells and outer root sheath cells was reduced upon treatment with Alda-1 (Example 5).

In yet another example of the present invention, since it was confirmed that hair follicles during the anagen phase had higher levels of ALDH2 and mitochondria and ALDH2 and mitochondria were present at the same location, it was confirmed that the interaction between ALDH2 and mitochondria is important for hair growth (Example 6).

In yet another example of the present invention, as a result of confirming changes in mitochondrial activity in outer root sheath cells according to the treatment with Alda-1, it was confirmed that the activation of ALDH2 may enhance the function of mitochondria, thereby contributing to the induction of the anagen phase of hair follicles (Example 7).

In yet another example of the present invention, as a result of confirming the combined effects of Alda-1 and minoxidil, it was confirmed that the combined administration of the two drugs can more effectively promote hair growth and hair follicle formation than treatment with either drug alone (Example 8).

Therefore, since the activation of ALDH2 may not only contribute to hair growth, but also achieve an excellent anti-gray hair effect by increasing the levels of hair growth-promoting factors and reducing oxidative stress to induce the proliferation of hair follicle cells, the activation of ALDH2 may be a therapy for the prevention, alleviation, and/or treatment of alopecia and gray hair. Furthermore, the activation of ALDH2 can exert a more synergistic alopecia treatment effect when used in combination with minoxidil, and thus may be usefully utilized as a combination therapy together with minoxidil for the prevention, alleviation, and/or treatment of alopecia.

Hereinafter, the present invention will be specifically described.

The present invention provides a pharmaceutical composition for preventing or treating alopecia or gray hair, including an aldehyde dehydrogenase 2 (ALDH2) activator as an active ingredient. In an exemplary embodiment of the present invention, the pharmaceutical composition (that is, an aldehyde dehydrogenase 2 activator) may be administered in combination with an alopecia therapeutic agent, but is not limited thereto.

In the present invention, "alopecia" refers to a state in which there is no hair in areas where hair should normally be present, and generally refers to the loss of the terminal hair (thick and black hair) of the scalp. When terminal hair falls out, it may cause cosmetic problems unlike vellus hair which is colorless and thin. Clinically, alopecia may be divided into two types: scarring and non-scarring, and in scarring alopecia, hair is not regenerated because hair follicles are destroyed, whereas in non-scarring alopecia, hair is regenerated after the affected area disappears because hair follicles are maintained. Noncicatricial alopecia, in which scars are not formed, includes hereditary androgenetic alopecia (baldness), alopecia areata, trichomycosis circinata caused by fungal infection, telogen effluvium, trichotillomania, hair production disorders, and the like, and cicatricial alopecia, in which scars are formed, includes alopecia caused by lupus, folliculitis decalvans, lichen planopilaris, alopecia caused by burns and trauma, and the like. The most common types of alopecia are hereditary androgenetic alopecia and alopecia areata, both of which do not cause scars. The most common alopecias include baldness (male pattern alopecia), female pattern alopecia, alopecia areata, telogen effluvium, and the like. The alopecia according to the present invention includes both male pattern alopecia and female pattern alopecia.

Preferably, the alopecia according to the present invention may be selected from the group consisting of alopecia areata, hereditary androgenetic alopecia, telogen effluvium, traumatic alopecia, trichotillomania, pressure alopecia, anagen alopecia, pityriasis alopecia, syphilitic alopecia, seborrheic alopecia, symptomatic alopecia, and congenital alopecia. In addition to the above diseases, the alopecia according to the present invention may include any problem caused by the phenomenon of large amounts of hair falling out, such as cosmetic problems. Preferably, the alopecia in the present invention is alopecia in the scalp.

The present invention may also be used to prevent and treat gray hair.

In the present invention, gray hair or white hair refers to hair (particularly scalp hair) that has lost its original color and turned gray or white due to aging, stress, or the like. The gray hair is a concept that includes not only gray hair occurring naturally during the aging process, but also gray hair occurring on the heads of young people who are not aging. Further, the gray hair includes not only a state in which the entire scalp hair has turned white, but also a state in which part of the scalp hair has turned white, and is a concept that includes not only the entire strand of hair turning white, but also part of a single hair turning white (that is, white stripes appearing in the hair). Although the molecular mechanisms responsible for gray hair are not clearly known, it is known that a reduction in function and number of melanocytes contributes to gray hair. In particular, since oxidative stress that occurs during the melanin production process in melanocytes damages melanocytes, it is particularly important to reduce the oxidative stress of hair follicles in order to prevent gray hair.

The gray hair includes poliosis. In the present invention, poliosis refers to a disease in which hair loses its color due to a reduction or disappearance of melanin in the hair. Poliosis may occur in adults as well as young children, and may be caused by genetic diseases, autoimmune diseases, damage to the hair follicles, or the like. The poliosis may occur not only in scalp hair but also in other body hair such as eyelashes and eyebrows. Preferably, the poliosis in the present invention is poliosis occurring in scalp hair.

In the present invention, aldehyde dehydrogenase (ALDH) is a group of enzymes that catalyze the oxidation of aldehydes. ALDH participates in a variety of biological processes including the detoxification of exogenous and endogenous aldehydes, and to date, 19 types of aldehyde dehydrogenase genes have been identified in the human genome.

In the present invention, aldehyde dehydrogenase 2 (ALDH2) is an enzyme of the subtype 2 of aldehyde dehydrogenase, and is encoded by the *ALDH2* gene located on chromosome 12. ALDH2 is present in mitochondria and converts acetaldehyde into acetic acid, thereby contributing to the breakdown of alcohol absorbed in the body. However, the effects of ALDH2 activation on alopecia or hair regrowth have not yet been reported. The ALDH2 protein and the gene encoding it have been widely known in the art, and may be typically identified in the protein database Uniprot (Accession No.: P05091).

The composition according to the invention may be for promoting hair growth and/or hair regrowth. As used herein, the term "hair regrowth" refers to the growth of new hair, "hair growth" refers to an increase in length of existing hair (that is, the growth of hair), and hair growth is used in the same sense as "hair care."

Throughout the present specification, hair includes both scalp hair (hair on the head) and body hair. Scalp hair refers to hair that grows on a person's scalp, and body hair includes hair that grows on parts of the body other than the scalp. Scalp hair and body hair differ in thickness, pore size, and the like, but as a representative example, in the case of scalp hair, 1 to 5 strands of hair per pore grow, whereas in the case of body hair, 1 strand of hair per pore mostly grows. Further, although there are differences depending on race, there are about 80,000 to 120,000 strands of hair per person. The thickness of scalp hair also varies from about 17 µm to 180 µm, and is usually characterized by being thicker than body hair, but the average thickness of scalp hair is known to be 80 µm to 150 µm, 90 µm to 150 µm, 100 µm to 150 µm, 110 µm to 150 µm, 120 µm to 150 µm, 130 µm to 150 µm, 80 µm to 140 µm, or 90 µm to 130 µm. Hair grows at different rates depending on the area where it grows, but for example, eyebrows, pubic hair, or the like grows about 0.05 to 0.2 mm per day, whereas scalp hair grows about 0.3 to 0.5 mm per day, and grows 1 cm or more based on one month. That is, scalp hair is characterized by growing faster than other body hair. Preferably, the composition according to the present invention is for promoting hair growth and/or hair regrowth.

In the present invention, an ALDH2 activator refers to a substance capable of promoting hair follicle growth, hair growth, and/or hair regrowth by enhancing the expression or activity of the ALDH2 protein. Further, the ALDH2 activator also includes a substance capable of preventing or alleviating gray hair by enhancing the expression or activity of the ALDH2 protein. Although not limited to a specific type, examples of the ALDH2 activator include an expression vector including an ALDH2 coding gene, an ALDH2 agonist, an ALDH2 function activator, and the like.

In the present invention, the expression of ALDH2 protein ultimately refers to the production of the ALDH2 protein, and enhancing the expression of the ALDH2 protein means including both increasing the expression of mRNA at the transcription stage of the ALDH2 coding gene and increasing the translation of the mRNA. In the present invention, an increase in expression of mRNA may involve the activation of a mechanism of action capable of increasing the transcription of the ALDH2 gene in a higher mechanism of action of the ALDH2 coding gene, or conversely, the inhibition of a higher mechanism of action capable of inhibiting the transcription of the ALDH2 gene, and further, the present invention includes all concepts that can directly or indirectly promote the expression of ALDH2, such as demethylation of the ALDH2 gene promoter region or histone modification associated with ALDH2 gene expression. In the present invention, increasing the translation of ALDH2 mRNA may include all mechanisms capable of enhancing the translation of the produced ALDH2 mRNA, and may be, for example, inhibiting the expression or activity of miRNA, and the like, which prevent the translation of ALDH2 mRNA, or increasing the activity of enzymes associated with the translation of ALDH2 mRNA, but is not limited thereto.

Further, in the present invention, "ALDH2 protein activity" refers to the activity of an ALDH2 protein produced through the translation process or the activity of an ALDH2 protein that has already been produced and present in cells, and "increasing the activity of the ALDH2 protein" includes any increase in ALDH2 protein activity through processes such as post-translational modifications or by the activity/inactivity of proteins, and the like which interact with it.

In the present invention, the aldehyde dehydrogenase 2 activator may include a substance capable of enhancing the expression or activity of the aldehyde dehydrogenase 2 protein without limitation, but may preferably be selected from the group consisting of Alda-1, AD-9308, compound 1-6, and pharmaceutically acceptable salts thereof.

In the present invention, the aldehyde dehydrogenase 2 activator may preferably be Alda-1. Alda-1 is a compound that is an agonist of ALDH2, and may permeate the cell membrane to activate the ALDH2 protein. Although Alda-1 is known to be able to reduce oxidative stress in liver cells and the like, there have been no reports on the effects of Alda-1 on hair follicles or hair growth. Through specific experiments using Alda-1, the present inventors have newly discovered that Alda-1 can be used to treat alopecia by promoting the growth of hair through the activation of ALDH2. Minoxidil, a potassium channel activator, promotes hair growth by hyperpolarizing cell membranes and expanding blood vessels in hair follicles to increase blood flow, but due to the strong vasodilatory properties of minoxidil, excessive use of minoxidil may rather have the risk of inducing fluid retention or edema formation. In contrast, it has been confirmed that Alda-1 according to the present invention can alleviate cell stress by removing cytotoxic aldehydes through the activation of ALDH2, and in particular, can inhibit oxidative stress in hair follicle cells, which is one of the main causes of alopecia. In particular, in an experiment using a mouse model of alopecia, the present inventors confirmed that Alda-1 exhibited the same or better alopecia treatment effect even when administered at a lower dose than minoxidil, and that the combined use of the two drugs can more effectively induce the growth of hair than using the drugs alone. That is, since Alda-1 can achieve excellent hair growth promoting effects even at low doses, it is possible to not only remarkably reduce the risk of side effects caused by excessive use of minoxidil, but also exhibit a synergistic effect in treating alopecia when used in combination with minoxidil. Furthermore, it has been confirmed that Alda-1 enhances mitochondrial function in hair follicles and remarkably reduces oxidative stress in hair follicles, so Alda-1 may also be used for preventing, alleviating, and treating gray hair or poliosis caused by oxidative stress.

Alda-1 according to the present invention may be referred to as N-(1,3-benzodioxol-5-ylmethyl)-2,6-dichlorobenzamide. Preferably, Alda-1 according to the present invention may have a structure of the following Chemical Formula 1, but is not limited thereto.

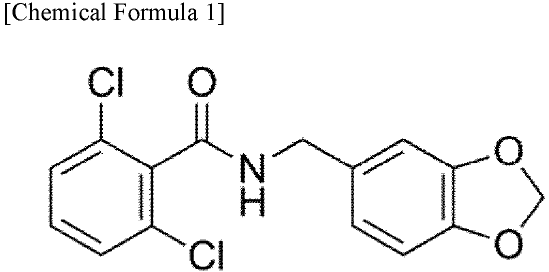

In the present invention, AD-9308 is a water-soluble ALDH2 activator, and is a more potent ALDH2 activator than Alda-1 which is a prototype. Recently, it has been reported that AD-9308 is effective in alleviating myocardial fibrosis, inflammation, and the like, and treating cardiac dysfunction, but the efficacy of AD-9308 in treating alopecia is unknown. Specific information on the chemical structure and properties of AD-9308, and the like can be found in the reference [Antioxidants (Basel). 2021 Mar 13; 10(3):450.] and U.S. Patent No. 9,879,036B2.

In the present invention, compound 1-6 refers to compound 1-6 disclosed in the reference [Journal of Saudi Chemical Society Volume 23, Issue 3, March 2019, Pages 255-262]. Compound 1-6 is a compound prepared by modifying Alda-1, and is an ALDH2 activator with higher water solubility and a higher ALDH2 activation effect. Specific information such as the chemical structure and properties of compound 1-6, and the like can be found in the aforementioned reference.

In the present invention, the alopecia therapeutic agent refers to a substance capable of directly or indirectly exhibiting a preventive or therapeutic effect on alopecia by promoting the formation and growth of hair follicles, inducing the anagen phase, relieving the stress on hair follicles, and the like. The alopecia therapeutic agent is sufficient if it exhibits an effect of preventing or treating alopecia by promoting hair regrowth or hair growth, and is not limited to a specific type, but may preferably be selected from minoxidil, finasteride, dutasteride, pharmaceutically acceptable salts thereof, analogs thereof, or the like. In a preferred embodiment, the alopecia therapeutic agent is minoxidil or a pharmaceutically acceptable salt thereof.

Minoxidil, also known as Rogaine, is a vasodilator used to treat alopecia, and its IUPAC ID is 6-piperidin-1-ylpyrimidine-2,4-diamine 3-oxide. Since the topical application of minoxidil increases the blood and nutrient supply to hair follicles, it is possible to strengthen existing hair and promote its growth. Although the molecular mechanism by which minoxidil promotes hair growth has not been specifically clarified, it is known that minoxidil induces hair growth by reversing the miniaturization of hair follicles, increasing blood flow around hair follicles, stimulating hair follicles to enter the anagen phase, and prolonging the anagen phase of hair follicles. However, it is known that when administered at a high dose, minoxidil may cause side effects such as rashes, inflammation, itching, weight gain, edema, and angina pectoris. Thus, an object of the present invention is to reduce the risk of side effects of minoxidil and provide a better alopecia treatment effect using an ALDH2 activator in combination with minoxidil. Specifically, by using an ALDH2 activator in combination with minoxidil, the present invention may, while using a relatively low concentration of minoxidil, exhibit alopecia prevention, hair growth, and hair regrowth promotion effects at the same or higher levels than when a standard amount of minoxidil is used to show the effects of preventing or treating alopecia. Throughout the present specification, "minoxidil" is a concept that includes not only minoxidil itself, but also salts thereof, analogs thereof, and the like.

In the present invention, minoxidil may be 0.1 to 10 (w/v)%, 0.1 to 8 (w/v)%, 0.1 to 6 (w/v)%, 0.1 to 5 (w/v)%, 0.1 to 3 (w/v)%, 1 to 10 (w/v)%, 1 to 8 (w/v)%, 1 to 6 (w/v)%, 1 to 5 (w/v)%, 1 to 3 (w/v)%, or 2 to 5 (w/v)% minoxidil. Alternatively, the minoxidil of the present invention may be 2 (w/v)%, 3 (w/v)%, or 5 (w/v)% minoxidil. In general, 5% or higher minoxidil is known to be particularly suitable for use by men, and lower concentrations such as 1 to 3% minoxidil are known to be able to be used by both men and women. The minoxidil may be dissolved in water, distilled water, deionized water, ultrapure water, or DMSO.

In the present invention, the ALDH2 activator includes not only the ALDH2 activator itself, but also all of pharmaceutically acceptable salts thereof, analogs thereof, derivatives thereof, and the like. For example, Alda-1 includes not only Alda-1 itself, but also pharmaceutically acceptable salts thereof, analogs thereof, derivatives thereof, and the like. The alopecia therapeutic agent of the present invention includes not only the alopecia therapeutic agent itself, but also pharmaceutically acceptable salts thereof, analogs thereof, derivatives thereof, and the like. For example, minoxidil is a concept that includes not only minoxidil itself, but also pharmaceutically acceptable salts thereof, analogs thereof, derivatives thereof, or the like.

In the present invention, the term "pharmaceutically acceptable salt" includes a salt derived from a pharmaceutically acceptable inorganic acid, organic acid, or base. Further, the derivatives include pharmaceutically acceptable salts, pharmaceutically acceptable solvates, pharmaceutically acceptable hydrates, pharmaceutically acceptable anhydrides, pharmaceutically acceptable enantiomers, pharmaceutically acceptable esters, pharmaceutically acceptable polymorphs, pharmaceutically acceptable prodrugs, pharmaceutically acceptable complexes, and the like.

Alternatively, as used herein, the term "pharmaceutically acceptable" refers to a compound or composition which is suitable for use in contact with tissues of a subject (for example, a human) and within the scope of the sound medical judgment because its benefit/risk ratio is reasonable without excessive toxicity, irritation, allergic reactions or other problems or complications.

Examples of a suitable acid include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, gluconic acid, naphthalene-2-sulfonic acid, benzenesulfonic acid, and the like. An acid addition salt may be prepared by a typical method, for example, dissolving a compound in an excess amount of an aqueous acid solution and precipitating the salt using a water-miscible organic solvent such as methanol, ethanol, acetone or acetonitrile. In addition, the acid addition salt may be prepared by heating equimolar amounts of a compound and an acid or alcohol in water, and then evaporating the mixture to dry the mixture, or suction-filtering the precipitated salt.

A salt derived from a suitable base may include an alkali metal such as sodium and potassium, an alkaline earth metal such as magnesium, ammonium and the like, but is not limited thereto. An alkali metal or alkaline earth metal salt may be obtained by, for example, dissolving the compound in an excess amount of an alkali metal hydroxide or alkaline earth metal hydroxide solution, filtering an undissolved compound salt, evaporating the filtrate, and drying the resulting product. In this case, it is particularly pharmaceutically suitable to prepare a sodium, potassium or calcium salt as the metal salt, and the corresponding silver salt may also be obtained by reacting an alkali metal or alkaline earth metal salt with a suitable silver salt (for example, silver nitrate).

The scope of the compound of the present invention includes not only pharmaceutically acceptable salts, but also all isomers, hydrates and solvates that can be prepared by typical methods.

The ALDH2 activator according to the present invention may be administered in combination with an alopecia therapeutic agent. The term co-administration used herein may be achieved by a method of administering the individual components of the treatment regimen simultaneously, sequentially or separately. The effect of combination therapy is obtained by a method of administering two or more drugs simultaneously or sequentially, or administering them alternately at regular or irregular intervals, and the combination therapy is not limited thereto, but may be defined as one that can provide a synergistic effect while having therapeutically better efficacy as measured, for example, through the degree of response, rate of response, period to disease progression or survival period, compared to the efficacy which may be obtained by administering one or the other of the components of the combination therapy at a typical dose. For example, the ALDH2 activator of the present invention, when administered in combination with minoxidil, may enhance the alopecia therapeutic effect of minoxidil or reduce the side effects caused by minoxidil. The administration (treatment) is a concept that includes all of injection, internal use, inhalation, spraying, application, and the like.

In the present invention, the ALDH2 activator may be administered simultaneously, separately, or sequentially with an alopecia therapeutic agent (for example, minoxidil), and even when the ALDH2 activator is administered sequentially with the alopecia therapeutic agent, the order of administration is not limited, but the administration regimen may be appropriately adjusted depending on the type of alopecia therapeutic agent, the patient's condition, the patient's sex, age, and the like.

The present invention also provides a pharmaceutical composition for preventing or treating alopecia, including an aldehyde dehydrogenase 2 activator and minoxidil as active ingredients.

The composition may be in the form of a mixture in which the ALDH2 activator and minoxidil are mixed, and may be in a form for simultaneous administration of the ALDH2 activator and minoxidil. For example, the ALDH2 activator and minoxidil may be a single formulation prepared by being mixed with a pharmaceutically acceptable adjuvant, diluent, carrier, or the like. In this case, the ALDH2 activator and minoxidil may each include various ingredients within the category of the corresponding drug. Further, the single formulation may be administered together with a separate preparation including another therapeutic agent as a separate formulation from the single formulation.

Alternatively, the composition may be in a form in which the ALDH2 activator and minoxidil are each formulated and administered simultaneously, separately, or sequentially. In this case, the composition may be a pharmaceutical composition for co-administration that is simultaneously or sequentially administered, including a first pharmaceutical composition including a pharmaceutically effective amount of the ALDH2 activator as an active ingredient; and a second pharmaceutical composition including a pharmaceutically effective amount of minoxidil as an active ingredient. In other words, the ALDH2 activator and minoxidil may be provided as separate pharmaceutical formulations, with each preparation being administered at the same time or at different times. The ALDH2 activator and minoxidil may be in the same formulation or in different formulations, and may be administered independently according to the formulation. In this case, in the case of sequential administration, the administration order is not limited, and the administration regimen may be appropriately adjusted depending on the condition of the patient, and the like.

For example, when the pharmaceutical composition is a pharmaceutical composition for co-administration that is sequentially administered, the ALDH2 activator (first component) may be administered first, and then the minoxidil (second component) may be administered, or the reverse order is also possible.

The content of the ALDH2 activator (for example, Alda-1), alopecia therapeutic agent (for example, minoxidil), and/or pharmaceutically acceptable salts thereof in the composition of the present invention can be appropriately adjusted depending on the symptoms of the disease, the degree of progression of the symptoms, the condition of the patient, and the like, and may be, for example, 0.0001 to 99.9 wt% or 0.001 to 50 wt% based on the total weight of the composition, but is not limited thereto. The content ratio is a value based on a dry amount from which the solvent is removed.

In an embodiment of the present invention, the ALDH2 activator (for example, Alda-1) or a pharmaceutically acceptable salt thereof may be contained at a concentration of 0.0001 to 50 mM with respect to the entire composition, but is not limited thereto. More specifically, Alda-1 or a pharmaceutically acceptable salt thereof according to the present invention may be contained in the composition at a concentration of 0.0001 to 50 mM, 0.0001 to 40 mM, 0.0001 to 30 mM, 0.0001 to 20 mM, 0.0001 to 10 mM, 0.0001 to 9 mM, 0.0001 to 8 mM, 0.0001 to 7 mM, 0.0001 to 6 mM, 0.0001 to 5 mM, 0.0001 to 4 mM, 0.0001 to 3 mM, 0.0001 to 2 mM, 0.0001 to 1 mM, 0.0005 to 10 mM, 0.001 to 10 mM, 0.005 to 10 mM, 0.01 to 10 mM, 0.05 to 10 mM, 0.1 to 10 mM, 0.5 to 10 mM, 1 to 30 mM, 1 to 20 mM, 1 to 15 mM, 1 to 10 mM, 1 to 9 mM, 1 to 8 mM, 1 to 7 mM, 1 to 6 mM, or 1 to 5 mM with respect to the entire composition, but is not limited thereto.

In other words, the composition of the present invention may include an ALDH2 activator or a pharmaceutically acceptable salt thereof in an amount such that the ALDH2 activator or a pharmaceutically acceptable salt thereof is administered (applied) at a concentration of 0.0001 to 50 mM with respect to the entire composition (or solvent) when administered or applied once to a subject in need thereof. That is, in the present invention, the ALDH2 activator or a pharmaceutically acceptable salt thereof may be administered (to a subject in need thereof) at a concentration of 0.0001 to 50 mM with respect to the entire composition (or solvent).

The composition according to the invention may be in the form of a liquid preparation. When the composition of the present invention is a liquid preparation, the ALDH2 activator or a pharmaceutically acceptable salt thereof may be included at a concentration of 0.0001 to 50 mM with respect to the entire liquid preparation. Further, the liquid preparation may be administered at a dose of 10 µL to 20 mL, 10 µL to 10 mL, 10 µL to 5 mL, 10 µL to 1 mL, 10 µL to 900 µL, 10 µL to 800 µL, 10 µL to 700 µl to 500 µl, 10 µl to 400 µL, 10 µl to 300 µL, 10 µL, or 10 µl to 100 µl when administered (applied) once, but is not limited thereto.

In an example of the present invention, the alopecia therapeutic agent (for example, minoxidil) or a pharmaceutically acceptable salt thereof may be contained at a concentration of 0.0001 to 10 (w/v)% with respect to the entire composition, but is not limited thereto. More specifically, the alopecia therapeutic agent or a pharmaceutically acceptable salt thereof according to the present invention may be contained in the composition at a concentration of 0.0001 to 10 (w/v)%, 0.0001 to 5 (w/v)%, 0.0001 to 3 (w/v)%, 0.0001 to 2 (w/v)%, 0.0001 to 1 (w/v)%, 0.0005 to 5 (w/v)%, 0.0005 to 3 (w/v)%, 0.0005 to 2 (w/v)%, 0.0005 to 1 (w/v)%, 0.001 to 5 (w/v)%, 0.001 to 3 (w/v)%, 0.001 to 2 (w/v)%, 0.001 to 1 (w/v)%, 0.005 to 5 (w/v)%, 0.005 to 3 (w/v)%, 0.005 to 2 (w/v)%, 0.01 to 5 (w/v)%, 0.01 to 3 (w/v)%, 0.01 to 2 (w/v)%, 0.05 to 2 (w/v)%, 0.1 to 2 (w/v)%, or 0.1 to 1 (w/v)%, but is not limited thereto.

Alternatively, the composition of the present invention may include an alopecia therapeutic agent or a pharmaceutically acceptable salt thereof in an amount such that the alopecia therapeutic agent or a pharmaceutically acceptable salt thereof is administered (applied) at a concentration of 0.0001 to 10 (w/v)% with respect to the entire composition (or solvent) when the composition is administered or applied once to a subject in need thereof. That is, in the present invention, the alopecia therapeutic agent or a pharmaceutically acceptable salt thereof may be administered (to a subject in need thereof) at a concentration of 0.0001 to 10 (w/v)% with respect to the entire composition (or solvent).

As described above, the composition according to the present invention may be in the form of a liquid preparation, and when the composition of the present invention is a liquid preparation, the alopecia therapeutic agent (for example, minoxidil) or a pharmaceutically acceptable salt thereof may be contained at a concentration of, but is not limited to, 0.0001 to 10 (w/v)% with respect to the entire liquid preparation. Further, the liquid preparation may be administered at a dose of 10 µL to 20 mL, 10 µL to 10 mL, 10 µL to 5 mL, 10 µL to 1 mL, 10 µL to 900 µL, 10 µL to 800 µL, 10 µL to 700 µl to 500 µl, 10 µl to 400 µL, 10 µl to 300 µL, 10 µL, or 10 µl to 100 µl when administered (applied) once, but is not limited thereto.

The ALDH2 activator and the alopecia therapeutic agent (for example, minoxidil, an analog thereof, or a pharmaceutically acceptable salt thereof) of the present invention may be used in combination at a weight ratio of 1:0.01 to 100, but is not limited thereto. Specifically, the ALDH2 activator and minoxidil may be used in combination at a weight ratio of 1 : 0.01 to 100, 1 : 0.01 to 80, 1 : 0.01 to 60, 1 : 0.01 to 50, 1 : 0.01 to 40, 1 : 0.01 to 30, 1 : 0.01 to 20, 1 : 0.01 to 10, 1 : 1 to 100, 1 : 1 to 80, 1 : 1 to 50, 1 : 1 to 20, 1 : 1 to 10, 1 : 10 to 100, 1 : 10 to 50, 1 : 10 to 40, 1 : 10 to 30, 1 : 20 to 100, 1 : 20 to 80, 1 : 20 to 50, 1 : 20 to 40, 1 : 30 to 100, 1 : 30 to 80, 1 : 30 to 50, 1 : 30 to 40, 1 : 35 to 40, or 1 : 37, but is not limited thereto.

Further, when the ALDH2 activator and the alopecia therapeutic agent of the present invention are used in combination in the form of a liquid preparation, each liquid preparation may be mixed together and treated in combination in the form of a mixed preparation. The mixed preparation may be prepared, for example, by mixing a liquid preparation including an ALDH2 activator at a concentration of 0.0001 to 50 mM with a liquid preparation including an alopecia therapeutic agent at a concentration of 0.0001 to 10 (w/v)%. Here, the liquid preparation including the ALDH2 activator and the liquid preparation including the alopecia therapeutic agent may be mixed at a volume ratio of 1 : 0.1 to 10, 1 : 0.1 to 5, 1 : 0.1 to 2, 1 : 0.1 to 1.5, 1 : 0.1 to 1, 1 : 0.5 to 5, 1 : 0.5 to 2, 1 : 0.5 to 1.5, or 1 : 1, but is not limited thereto. Further, the mixed preparation may be administered at a dose of 10 µL to 20 mL, 10 µL to 10 mL, 10 µL to 5 mL, 10 µL to 1 mL, 10 µL to 900 µL, 10 µL to 800 µL, 10 µL to 700 µl to 500 µl, 10 µl to 400 µL, 10 µl to 300 µL, 10 µL, or 10 µl to 100 µl upon administration (application or treatment) once, but is not limited thereto.

The treatment concentration, treatment dose, and the like of the ALDH2 activator and alopecia therapeutic agent described above may be particularly suitable when the pharmaceutical composition according to the present invention is applied on the skin, and the like of a subject in need thereof. However, the administration form of the pharmaceutical composition according to the present invention is not limited to a specific type, and a person skilled in the art may select an appropriate volume, concentration, mixing ratio, and the like, in consideration of the drug treatment route, treatment method, types of drugs used in combination, the condition of the subject, and the like based on common technical knowledge in the art.

Preferably, the composition according to the present invention may satisfy one or more characteristics selected from the group consisting of, but not limited to: (a) promoting the proliferation (cell division) of hair dermal papilla cells and/or outer root sheath cells; (b) promoting the growth of hair follicles (that is, promoting the growth or cell division of cells in hair follicles); (c) inducing the anagen phase of hair; and (d) increasing mitochondrial activity.

Preferably, the composition of the present invention may increase the level or activity of one or more proteins selected from the group consisting of basic fibroblast growth factor (bFGF), keratinocyte growth factor (FGF-7), insulin-like growth factor 1 (IGF-1), and platelet-derived growth factor (PDGF), but is not limited thereto.

Preferably, the composition according to the present invention may reduce oxidative stress in hair dermal papilla cells or outer root sheath cells, but is not limited thereto.

The pharmaceutical composition of the present invention may further include an appropriate carrier, excipient, and diluent, which are typically used to prepare a pharmaceutical composition. The excipient may be, for example, one or more selected from the group consisting of a diluent, a binder, a disintegrant, a lubricant, an adsorbent, a moisturizer, a film-coating material, and a controlled release additive.

The pharmaceutical composition according to the present invention may be used by being formulated into the form of a powder, a granule, a sustained-release granule, an enteric granule, a liquid, a collyrium, an elixir, an emulsion, a suspension, a spirit, a troche, aromatic water, a limonade, a tablet, a sustained-release tablet, an enteric tablet, a sublingual tablet, a hard capsule, a soft capsule, a sustained-release capsule, an enteric capsule, a pill, a tincture, a soft extract agent, a dry extract agent, a fluid extract agent, an injection, a capsule, a perfusate, an external preparation such as a plaster, a lotion, a paste, a spray, an inhalant, a patch, a sterilized injection solution, or an aerosol, and the external preparation may have a formulation such as a cream, a gel, a patch, a spray, an ointment, a plaster, a lotion, a liniment, a paste or a cataplasma.

Examples of a carrier, an excipient or a diluent which may be included in the composition according to the present invention include lactose, dextrose, sucrose, an oligosaccharide, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

When the pharmaceutical composition is prepared, the pharmaceutical composition is prepared using a diluent or excipient, such as a filler, an extender, a binder, a wetting agent, a disintegrant, and a surfactant, which are commonly used.

As an additive of the tablet, powder, granule, capsule, pill, and troche according to the present invention, it is possible to use an excipient such as corn starch, potato starch, wheat starch, lactose, white sugar, glucose, fructose, D-mannitol, precipitated calcium carbonate, synthetic aluminum silicate, calcium monohydrogen phosphate, calcium sulfate, sodium chloride, sodium hydrogen carbonate, purified lanolin, microcrystalline cellulose, dextrin, sodium alginate, methyl cellulose, carboxymethyl cellulose sodium, kaolin, urea, colloidal silica gel, hydroxypropyl starch, hydroxypropyl methylcellulose (HPMC) 1928, HPMC 2208, HPMC 2906, HPMC 2910, propylene glycol, casein, calcium lactate, and Primojel; and a binder such as gelatin, arabic gum, ethanol, agar powder, cellulose acetate phthalate, carboxymethyl cellulose, carboxymethyl cellulose calcium, glucose, purified water, sodium caseinate, glycerin, stearic acid, carboxymethyl cellulose sodium, methylcellulose sodium, methylcellulose, microcrystalline cellulose, dextrin, hydroxycellulose, hydroxypropyl starch, hydroxymethyl cellulose, purified shellac, starch, hydroxypropyl cellulose, hydroxypropyl methyl cellulose, polyvinyl alcohol, and polyvinylpyrrolidone, and it is possible to use a disintegrant such as hydroxypropyl methyl cellulose, corn starch, agar powder, methyl cellulose, bentonite, hydroxypropyl starch, carboxymethyl cellulose sodium, sodium alginate, carboxymethyl cellulose calcium, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, arabic gum, amylopectin, pectin, sodium polyphosphate, ethyl cellulose, white sugar, magnesium aluminum silicate, a D-sorbitol solution, and light anhydrous silicic acid; and a lubricant such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubriwax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid.

As an additive for the liquid formulation according to the present invention, it is possible to use water, diluted hydrochloric acid, diluted sulfuric acid, sodium citrate, sucrose monostearate, polyoxyethylene sorbitol fatty acid esters (Tween esters), polyoxyethylene monoalkyl ethers, lanolin ether, lanolin esters, acetic acid, hydrochloric acid, aqueous ammonia, ammonium carbonate, potassium hydroxide, sodium hydroxide, prolamin, polyvinyl pyrrolidone, ethyl cellulose, carboxymethyl cellulose sodium, and the like.

In the syrup according to the present invention, a solution of sucrose, other sugars or sweeteners, and the like may be used, and a fragrance, a colorant, a preservative, a stabilizer, a suspending agent, an emulsifier, a thickener, and the like may be used if necessary.

Purified water may be used for the emulsion according to the present invention, and an emulsifier, a preservative, a stabilizer, a fragrance, and the like may be used if necessary.

In the suspension according to the present invention, a suspending agent such as acacia, tragacanth, methyl cellulose, carboxymethyl cellulose, carboxymethyl cellulose sodium, microcrystalline cellulose, sodium alginate, hydroxypropyl methyl cellulose (HPMC), HPMC 1828, HPMC 2906, and HPMC 2910 may be used, and a surfactant, a preservative, a colorant, and a fragrance may be used, if necessary.

The injection according to the present invention may include a solvent such as distilled water for injection, 0.9% sodium chloride injection, Ringer's injection, dextrose injection, dextrose+sodium chloride injection, PEG, lactated Ringer's injection, ethanol, propylene glycol, non-volatile oil-sesame oil, cottonseed oil, peanut oil, corn oil, ethyl oleate, isopropyl myristate, or benzoic acid benzene; a solubilizing aid such as sodium benzoate, sodium salicylate, sodium acetate, urea, urethane, monoethyl acetamide, butazolidin, propylene glycol, Tweens, niacinamide, hexamine, or dimethylacetamide; a buffer such as a weak acid or salt thereof (acetic acid or sodium acetate), a weak base or salt thereof (ammonia or ammonium acetate), an organic compound, a protein, albumin, peptone, or a gum; an isotonic agent such as sodium chloride; a stabilizer such as sodium bisulfite (NaHSO₃), carbon dioxide gas, sodium metabisulfite (Na₂S₂O₅), sodium sulfite (Na₂SO₃), nitrogen gas (N₂), or ethylenediaminetetraacetic acid; a sulfating agent such as 0.1% sodium bisulfide, sodium formaldehydesulfoxylate, thiourea, disodium ethylenediaminetetraacetate, or acetone sodium bisulfite; an analgesic such as benzyl alcohol, chlorobutanol, procaine hydrochloride, glucose, or calcium gluconate; and a suspending agent such as carboxymethyl cellulose sodium, sodium alginate, Tween 80, or aluminum monostearate.

A solid formulation for oral administration includes a tablet, a pill, a powder, a granule, a capsule, or the like, and the solid formulation is prepared by mixing at least one excipient, for example, starch, calcium carbonate, sucrose or lactose, gelatin, and the like, with an extract. Further, in addition to a simple excipient, lubricants such as magnesium stearate and talc are also used.

A liquid formulation for oral administration corresponds to a suspension, a liquid for internal use, an emulsion, a syrup, or the like, and the liquid formulation may include, in addition to water and liquid paraffin, which are simple commonly used diluents, various excipients, for example, a wetting agent, a sweetener, a fragrance, a preservative, and the like. Examples of a formulation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like.

The pharmaceutical composition according to the present invention is administered in a pharmaceutically effective amount. In the present invention, "pharmaceutically effective amount" means an amount sufficient to treat diseases at a reasonable benefit/risk ratio applicable to medical treatment, and an effective dosage level may be determined according to factors including the type of disease of a patient, the severity of the disease, the activity of drugs, sensitivity to drugs, administration time, administration route, excretion rate, treatment period, and simultaneously used drugs, and other factors well known in the medical field. For example, the composition according to the present invention may be administered (applied) to a subject in need thereof 1 to 5 times, 1 to 3 times, 1 to 2 times, or once per day. In this case, the dosage may be adjusted according to the severity of the alopecia-related lesion.

The pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this amount may be easily determined by a person with ordinary skill in the art to which the present invention pertains.

Further, the pharmaceutical composition according to the present invention may be administered to a subject in need thereof 1 to 10 times, 1 to 5 times, 1 to 3 times, or 1 to 2 times per day, and may be administered at intervals of 1 to 10 days, 1 to 5 days, 1 to 3 days, or every day, but is not limited thereto.

The pharmaceutical composition of the present invention may be administered to a subject in need via various routes. All methods of administration may be contemplated, but the pharmaceutical composition may be administered by, for example, oral administration, subcutaneous injection, peritoneal administration, intravenous injection, intramuscular injection, paraspinal space (intradural) injection, sublingual administration, buccal administration, intrarectal insertion, intravaginal injection, ocular administration, ear administration, nasal administration, inhalation, spraying via the mouth or nose, skin administration, transdermal administration, and the like.

The pharmaceutical composition of the present invention is determined by the type of drug that is an active ingredient, as well as various related factors such as the disease to be treated, the route of administration, the age, sex, and body weight of a patient, and the severity of the disease.

As used herein, the "subject" refers to a subject in need of treatment of a disease, and more specifically, refers to a mammal such as a human or a non-human primate, a mouse, a rat, a dog, a cat, a horse, and a cow.

As used herein, "administration" refers to the provision of a predetermined composition of the present invention to a subject in need thereof by any suitable method.

As used herein, "prevention" refers to all actions that suppress or delay the onset of a target disease, and "treatment" refers to all actions that ameliorate or beneficially change a target disease and the resulting metabolic abnormalities by administration of the pharmaceutical composition according to the present invention, and "alleviation" refers to all actions that reduce a target disease and associated parameters, for example, the severity of symptoms, by administration of the composition according to the present invention.

Further, the present invention provides a cosmetic composition for preventing or alleviating alopecia or gray hair, including an aldehyde dehydrogenase 2 activator as an active ingredient. The cosmetic composition may be administered in combination with an alopecia therapeutic agent. Further, the present invention provides a cosmetic composition for preventing or alleviating alopecia, including an aldehyde dehydrogenase 2 activator and an alopecia therapeutic agent as active ingredients.

Preferably, the ALDH2 activator is Alda-1, an analog thereof, or a cosmetically acceptable salt thereof, and the alopecia therapeutic agent may be minoxidil, an analog thereof, or a cosmetically acceptable salt thereof.

Here, the prevention or alleviation of gray hair means suppressing the bleaching of hair to maintain its original color, and such an effect may be achieved particularly by maintaining the function and number of melanocytes.

A formulation of the cosmetic composition according to the present invention may be in the form of a skin lotion, skin softener, skin toner, astringent, lotion, milk lotion, moisturizing lotion, nourishing lotion, massage cream, nourishing cream, mist, moisturizing cream, hand cream, hand lotion, foundation, essence, nourishing essence, pack, soap, cleansing foam, cleansing lotion, cleansing cream, cleansing oil, cleansing balm, body lotion or body cleanser.

Preferably, the composition may be a formulation for external use in the form of a scalp treatment agent, soap, hair tonic, shampoo, rinse, hair pack, hair gel, lotion, conditioner, hair oil, mousse, cream, a solid agent, a solution, an emulsion, a dispersant, a micelle, a liposome, an ointment, toilet water, an essence, a patch, or a spray.

The cosmetic composition of the present invention may further include a composition selected from the group consisting of water-soluble vitamins, oil-soluble vitamins, polymer peptides, high molecular weight polysaccharides, and sphingolipids.

Other ingredients typically incorporated into cosmetics may also be blended with the cosmetic composition of the present invention, if necessary, together with the essential ingredients.

Examples of other blended ingredients which may be added include oil and fat ingredients, a moisturizer, an emollient, a surfactant, organic and inorganic pigments, an organic powder, an ultraviolet absorbent, a preservative, a bactericide, an antioxidant, a plant extract, a pH adjuster, an alcohol, a colorant, a fragrance, a circulation accelerator, a cooling agent, an antiperspirant, purified water, and the like.

Further, the blended ingredients that may be added are not limited to the aforementioned ingredients, and any ingredient can be blended within a range that does not impair the purpose and effect of the present invention, but may be blended at 0.01 to 5 wt%, or 0.01 to 3 wt% based on the total weight.

When the formulation of the present invention is a lotion, a paste, a cream, or a gel, animal fiber, vegetable fiber, a wax, paraffin, starch, tragacanth, a cellulose derivative, polyethylene glycol, silicone, bentonite, silica, talc, zinc oxide, or the like may be used as a carrier ingredient.

When the formulation of the present invention is a powder or a spray, lactose, talc, silica, aluminum hydroxide, calcium silicate, or a polyamide powder may be used as a carrier ingredient, and in particular, when the formulation is a spray, the formulation may include a propellent such as chlorofluorohydrocarbon, propane/butane or dimethyl ether.

When the formulation of the present invention is a solution or an emulsion, a solvent, a solubilizer or an emulsifier is used as a carrier ingredient, and examples thereof include water, ethanol, isopropanol, ethyl carbonate, ethyl acetate, benzyl alcohol, benzyl benzoate, propylene glycol, 1,3-butylglycol oil, glycerol aliphatic esters, polyethylene glycol or fatty acid esters of sorbitan.

When the formulation of the present invention is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, or the like may be used as a carrier ingredient.

When the formulation of the present invention is a surfactant-containing cleanser, an aliphatic alcohol sulfate, an aliphatic alcohol ether sulfate, sulfosuccinic acid monoester, isethionate, an imidazolinium derivative, methyltaurate, sarcosinate, fatty acid amide ether sulfate, alkylamidobetaine, an aliphatic alcohol, fatty acid glyceride, fatty acid diethanolamide, a vegetable oil, a lanolin derivative, an ethoxylated glycerol fatty acid ester, or the like may be used as a carrier ingredient.

Further, the composition according to the present invention may be provided in the form of a quasi-drug composition. In the present invention, "quasi-drug" refers to a product used for the purpose of treating, mitigating, managing, or preventing a disease in humans or animals, and a product which has only a slight effect on the human body or does not act directly on the human body.

The quasi-drug composition of the present invention may further contain, in addition to the aforementioned components, a pharmaceutically acceptable carrier, excipient, or diluent, if necessary. The pharmaceutically acceptable carrier, excipient, or diluent is not limited as long as it do not affect the effects of the present invention, and may include, for example, a filler, an extender, a binder, a wetting agent, a disintegrant, a surfactant, a lubricant, a sweetener, a fragrance, a preservative, and the like.

Representative examples of acceptable carriers, excipients, or diluents for the quasi-drug composition of the present invention include lactose, dextrose, sucrose, sorbitol, mannitol, xylitol, maltitol, starch, gelatin, glycerin, acacia rubber, alginate, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, mineral oil, propylene glycol, polyethylene glycol, vegetable oil, injectable ester, Witepsol, Macrogol, Tween 61, cacao butter, laurin butter, and the like.

When the composition of the present invention is provided as an external preparation, the composition may be in the form of, but is not limited to, a liquid, an ointment, a patch, a gel, a cream, or a spray. According to an exemplary embodiment of the present invention, the quasi-drug of the present invention may include an oral care product including toothpaste, mouthwash, and mouth spray, an ointment, a mask, a poultice, a patch, a transdermal agent, and the like. The method of formulation, dose, method of use, components, and the like of the quasi-drug may be appropriately selected from typical techniques known in the art.

Further, the present invention provides a food composition for preventing or alleviating alopecia or gray hair, including an aldehyde dehydrogenase 2 activator as an active ingredient. The composition may be administered in combination with an alopecia therapeutic agent. Further, the present invention provides a food composition for preventing or alleviating alopecia, including an ALDH2 activator and an alopecia therapeutic agent as active ingredients. Preferably, the alopecia therapeutic agent is minoxidil, an analog thereof, or a sitologically acceptable salt thereof.

The aldehyde dehydrogenase 2 activator may preferably be Alda-1 or a sitologically acceptable salt thereof. In the present invention, the sitologically acceptable salt includes a salt derived from a sitologically acceptable organic acid, inorganic acid, or base.

When ALDH2 activator of the present invention is used as a food additive, the ALDH2 activator may be added as is or used with another food or other food ingredients, and may be appropriately used according to a typical method. The amount of active ingredient mixed may be suitably determined according to the purpose of use (prevention, health or therapeutic treatment). In general, when a food or beverage is prepared, the ALDH2 activator of the present invention may be added in an amount of 15 wt% or less, or 10 wt% or less based on the raw materials. However, in the case of long-term intake for the purpose of health and hygiene or for the purpose of controlling health, the amount may be less than the above range, and the effective ingredient may be used in an amount above the above range because there is no problem in terms of safety.

The type of food is not particularly limited. Examples of food to which the material may be added include meats, sausage, bread, chocolate, candy, snacks, confectioneries, pizza, instant noodles, other noodles, gum, dairy products including ice cream, various soups, beverages, tea, drinks, alcoholic beverages, vitamin complexes, and the like, and include all health functional foods in a typical sense.

The health beverage composition according to the present invention may contain various flavors or natural carbohydrates and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin or aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combination. The proportion of these additives is not very important, but is generally selected within a range of 0.01 to 0.20 parts by weight per 100 parts by weight of the composition of the present invention.

As used herein, the health functional food is the same term as a food for special health use (FoSHU), and refers to a food having high medical and medicinal effects that is processed to efficiently exhibit bioregulatory functions, in addition to nutritional supply, and the food may be prepared in various forms such as tablets, capsules, powders, granules, liquids and pills, to obtain a useful effect in preventing or alleviating alopecia.

The health functional food can be prepared by a method typically used in the art, and may be prepared by adding raw materials and components typically added in the art during preparation. Furthermore, the health functional food has an advantage of having no side effects which may occur when the drug is taken for a long period of time because food is used as a raw material unlike general drugs, and may have excellent portability.

Hereinafter, preferred examples for helping with understanding of the present invention will be suggested. However, the following examples are provided only so that the present invention may be more easily understood, and the content of the present invention is not limited by the following examples.

### [Examples]

### Example 1. Induction of cell division and activation of hair dermal papilla cells and outer root sheath cells derived from human hair follicles by ALDH2 activation

To evaluate the effect of an ALDH2 activator on the proliferation of hair follicle cells, hair dermal papilla cells (HDPCs) and outer root sheath cells (ORSs) derived from human hair follicles were treated with an ALDH2 activator Alda-1 or a known alopecia therapeutic agent minoxidil at a concentration of 1 µM, and CCK8 assay was performed. Primary cultured hair dermal papilla cells were treated with Alda-1 at a concentration of 0, 25, 50, or 100 µM and cultured for 24 or 48 hours, and then the cells were treated with CCK8 reagent and allowed to react at 37°C for 15 minutes. Thereafter, the absorbance was measured at 450 nm using a luminometer, and the degree of cell proliferation was confirmed by comparing the cells with a negative control and a positive control, minoxidil.

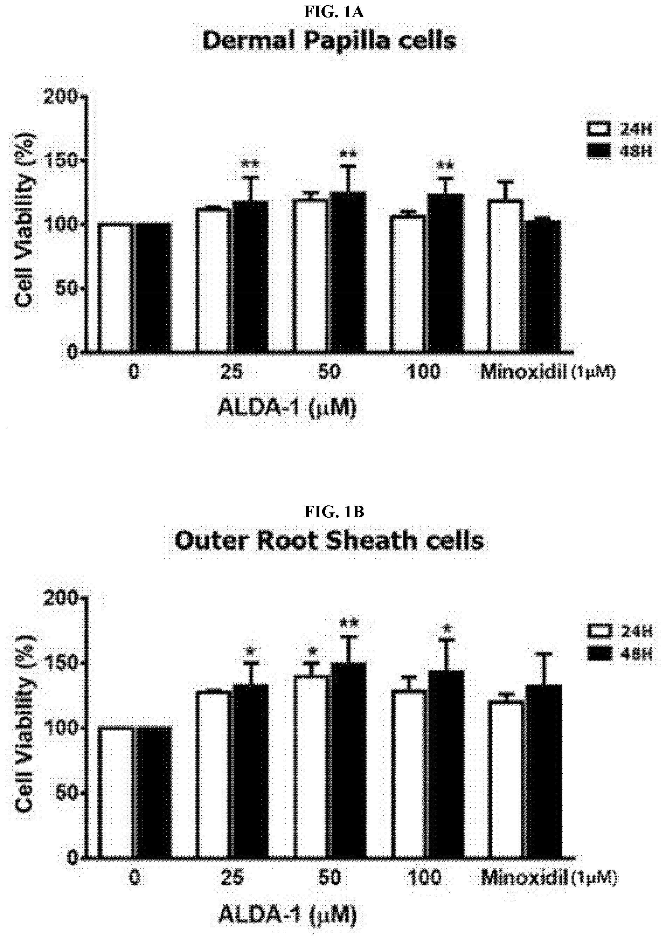

As a result, it was confirmed that treatment with Alda-1 significantly increased the cell viability of hair dermal papilla cells (FIG. 1A) and outer root sheath cells (FIG. 1B). In particular, Alda-1 was found to promote the proliferation of hair dermal papilla cells and outer root sheath cells at levels equivalent to or greater than those of minoxidil. These results support that the activation of ALDH2 may promote the proliferation of hair dermal papilla cells and outer root sheath cells, and exhibit an effect of promoting hair regrowth through this.

### Example 2. Confirmation of effect of ALDH2 activation on promoting human hair follicle growth through human hair follicle organ culture test

To evaluate the effect of ALDH2 activation on human hair follicle growth, a human hair follicle organ culture (*ex vivo* hair follicle organ culture) test was performed. To perform the human hair follicle organ culture test, scalp tissue obtained from a voluntary donor was separated into units of hair follicles, and a portion below the sebaceous gland of each separated hair follicle was cut off and the remaining hair follicles were used. After the hair follicles were cultured by adding a culture medium containing a negative control reagent; 1 µM minoxidil; or 5, 25, or 50 µM Alda-1 (ALDH2 activator) to the prepared hair follicles, the length of the growing hair was measured on days 3 and 6 and compared with the negative control to confirm the effect of the ALDH2 activator on promoting hair growth in human hair follicles.

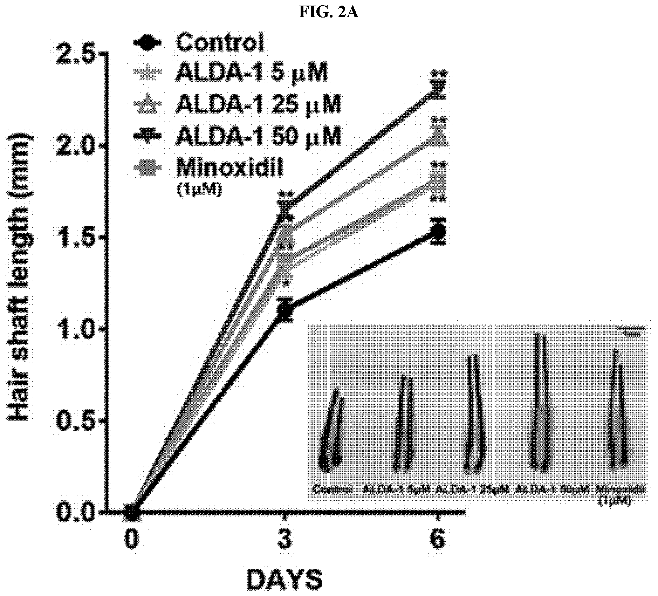

As a result, as shown in FIG. 2A, it could be confirmed that the activation of ALDH2 in the Alda-1-treated group promoted the elongation of hair in human hair follicles. In particular, Alda-1 was found to promote the growth of hair in hair follicles at higher levels compared to minoxidil.

Further, to confirm the proliferation of cells in the cultured hair follicles through the test, immunofluorescence staining was performed using a Ki-67 antibody, a cell division marker. Hair follicles were treated with the negative control, 1 µM minoxidil, or Alda-1 and cultured for 2 days to prepare tissue sections, the tissue sections were cut into a thickness of 4 µm and fixed on slides, and then treated with Ki-67 as a primary antibody and a fluorescent protein-conjugated secondary antibody to detect Ki-676, and then then observed under a fluorescent microscope and photographed to confirm the number of Ki-67 positive cells. Nuclei were stained using DAPI reagent.

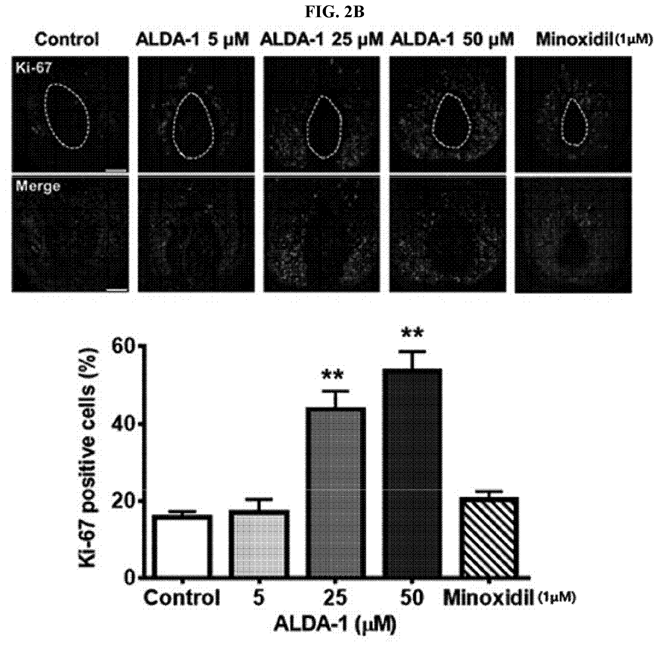

The results are shown in FIG. 2B (Ki-67, red fluorescence; and DAPI, blue fluorescence). As can be seen in FIG. 2B, as ADLH2 was activated in hair follicles treated with Alda-1, the level of Ki-67 positive cells, that is, cells undergoing division (proliferation) in the hair follicles was increased, and such an effect of Alda-1 was found to be even better than that of minoxidil.

The above results indicate that the activation of ALDH2 may promote the proliferation of cells in human hair follicles, thereby promoting hair growth.

### Example 3. Confirmation of effect of ALDH2 activation on inducing anagen phase of hair growth

To confirm the effect of ALDH2 activation on hair growth, the anagen-inducing effect of hair growth was confirmed using a C57BL/6 mouse model. Although human hair has different hair cycles depending on the individual, in mice, all hairs have the same cycle in the early stages of their lives, and at 7 to 8 weeks of age, all hair enters the telogen phase and then transitions to the anagen phase. To evaluate the effect of the ALDH2 activator on inducing the anagen phase using these characteristics of mice, hair was removed from the backs of 8-week-old C57BL/6 mice in the telogen state, and then the ALDH2 activator Alda-1 was applied at a dose of 0.5 mM or 3 mM once a day for three weeks, and the presence or absence of hair growth and the degree of inducing the anagen phase were confirmed by comparing with a negative control and a positive control (minoxidil 2 wt%; 111 mM).

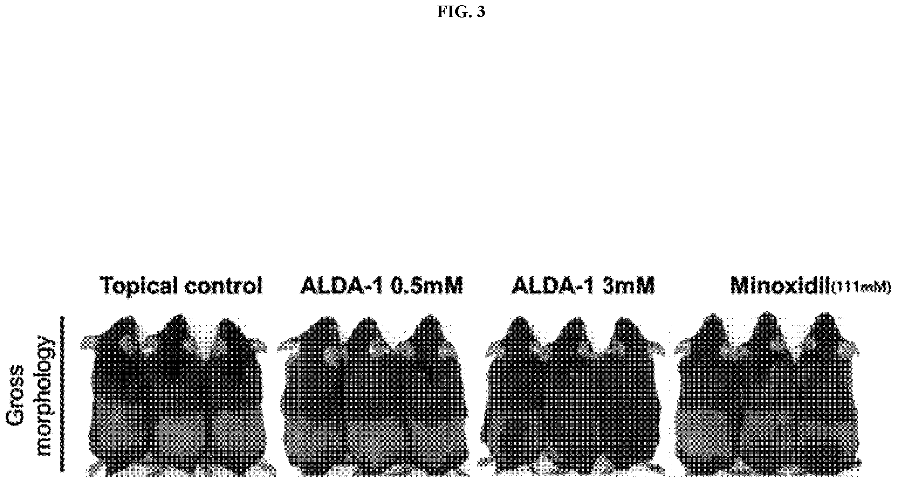

As a result, as shown in FIG. 3, the percentage of areas where hair in the anagen phase grew was significantly increased in the Alda-1 treated group, confirming that the activation of ALDH2 has an effect of promoting the induction of the anagen phase of hair. In particular, the Alda-1-treated group showed a similar or higher level of hair growth compared to the minoxidil-treated group, despite being treated with a much lower dose of the drug.

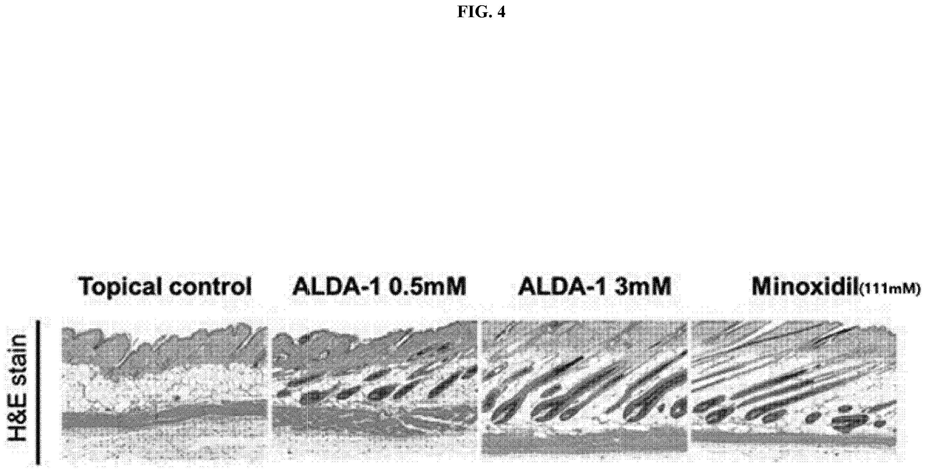

Further, skin tissue in which hair during the anagen stage grew was fixed in paraffin and stained with hematoxylin and eosin to perform a histological examination. As a result, as shown in FIG. 4, it could be confirmed that the formation of hair follicles was also promoted in the Alda-1-treated group at levels similar to those of the positive control treated with minoxidil.

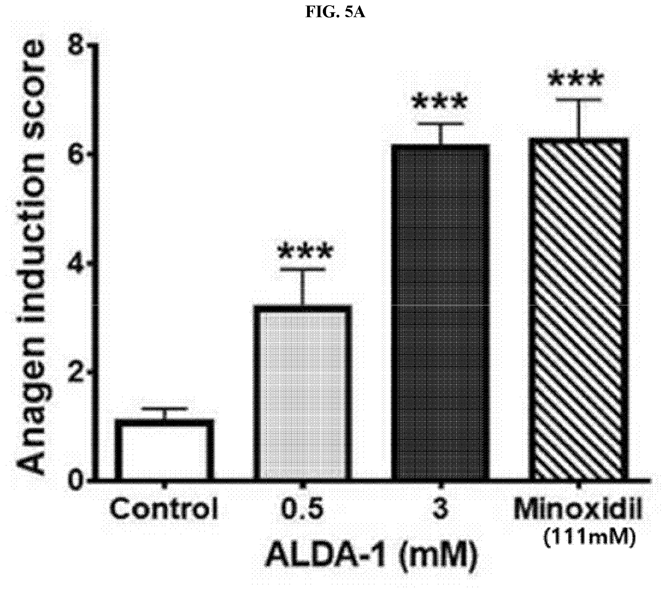

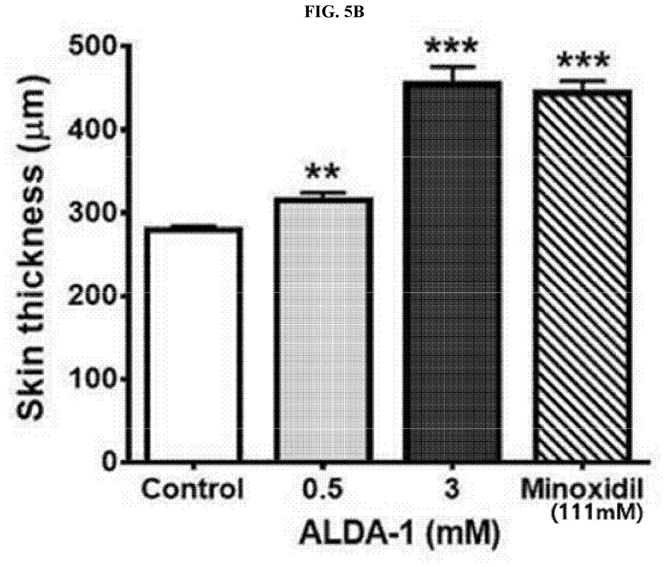

Furthermore, as a result of measuring the anagen induction level and skin thickness of each mouse, it was found that the anagen induction score and skin thickness of the ALDH2 activator-treated group were increased compared to the control, as shown in FIGS. 5A and 5B.

The above results indicate that the activation of ALDH2 promotes hair growth by inducing the anagen phase of hair.

### Example 4. Confirmation of hair growth promoting protein regulation effect by ALDH2 activation

To confirm the molecular biological mechanism by which the activation of ALDH2 promotes the proliferation of hair dermal papilla cells and outer root sheath cells and induces the anagen phase of hair follicles, changes in the protein levels of hair growth-promoting factors caused by the activation of ALDH2 were confirmed using human hair dermal papilla cells. For this purpose, an enzyme-linked immunosorbent assay (ELISA) method was performed. After hair dermal papilla cells cultured in a laboratory were treated with a negative control; or an ALDH2 activator Alda-1 for 24 hours, a cell culture medium was collected and concentrated. The levels of growth-promoting proteins were confirmed in the concentrated culture medium.

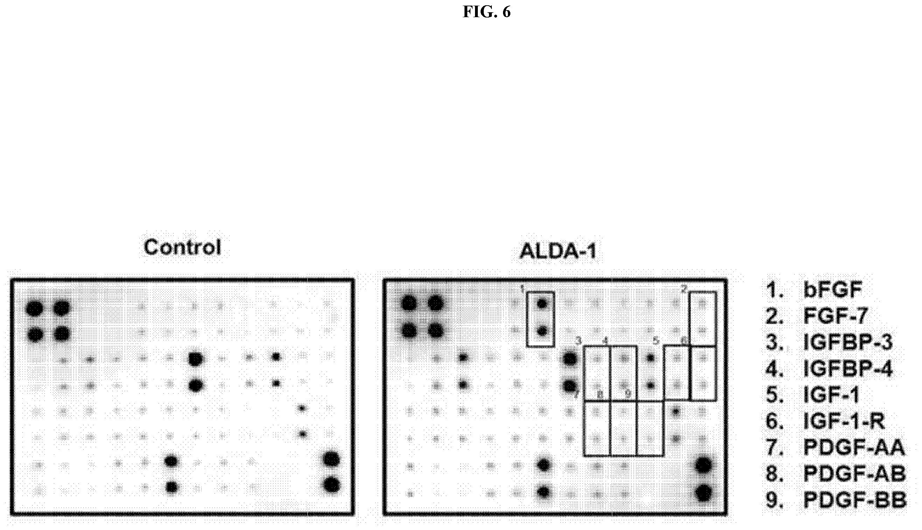

As a result, as shown in FIG. 6, it could be confirmed that in the samples treated with the ALDH2 activator, the levels of proteins such as basic fibroblast growth factor (bFGF), keratinocyte growth factor (FGF-7), insulin-like growth factor 1 (IGF-1), and platelet-derived growth factor (PDF) known as hair growth-promoting factors were increased.

The above results indicate that the activation of ALDH2 increases the protein levels of hair growth-promoting factors to promote the proliferation of cells associated with hair growth and induce the anagen phase of hair follicles.

### Example 5. Confirmation of reduction in oxidative stress in hair dermal papilla cells and outer root sheath cells derived from human hair follicles by ALDH2 activation

Oxidative stress in hair follicle cells is known to inhibit hair follicle and hair growth and adversely affect alopecia. Therefore, to evaluate the effect of ALDH2 activation on oxidative stress in hair follicle cells, hair dermal papilla cells and outer root sheath cells derived from human hair follicles were treated with the ALDH2 activator Alda-1, and 2',7'-dichlorofluorescin diacetate (DCFDA) assay was performed to confirm the degree of oxidative stress. The cultured cells of each group were treated with H₂O₂ to increase the oxidative stress level, and the experimental group was treated with the ALDH2 activator Alda-1 at a concentration of 5, 25, or 50 µM for 3 hours to observe the expression of DCFDA. Further, the same experiment was performed to confirm the expression level of DCFDA through flow cytometry.

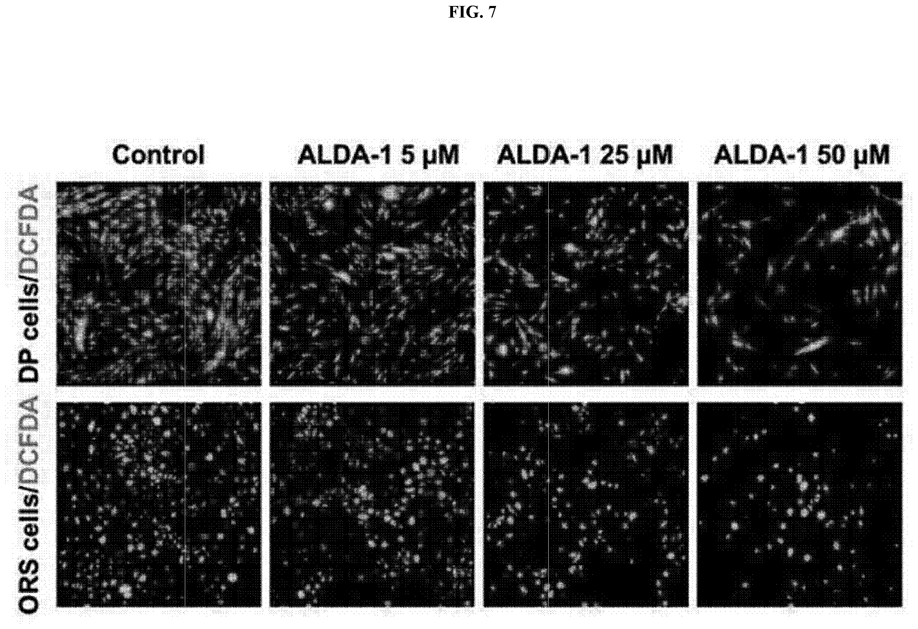

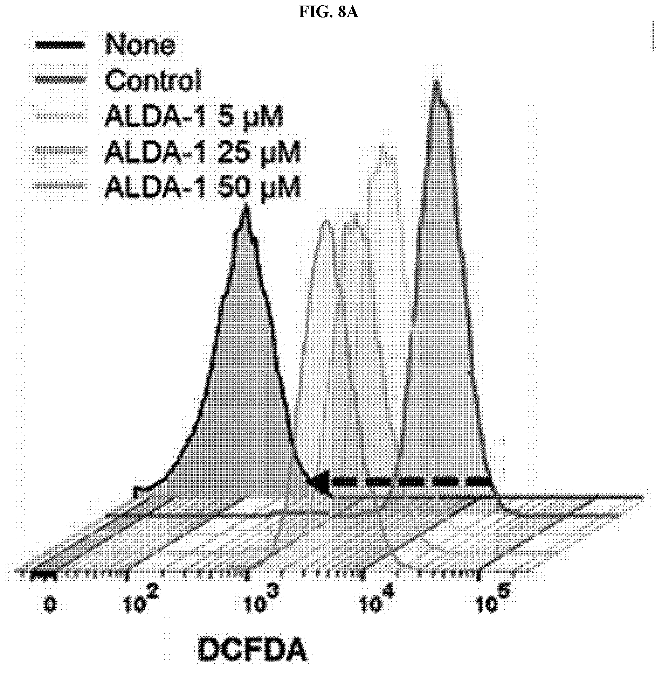

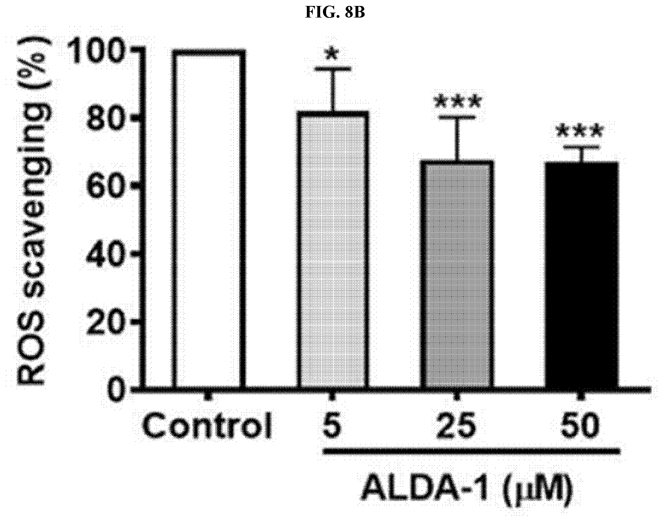

As a result, as shown in FIG. 7, the fluorescence level indicating DCFDA was significantly reduced in the Alda-1-treated group compared to the untreated control, confirming that oxidative stress was reduced by the ALDH2 activator. In particular, the oxidative stress was shown to decrease in proportion to the treatment concentration of Alda-1, confirming that the ALDH2 activator is highly effective in reducing oxidative stress in hair follicle cells. The flow cytometry results also showed that oxidative stress in outer root sheath cells was significantly reduced in proportion to the treatment concentration of Alda-1 (FIGS. 8A and 8B).

The above results indicate that the activation of ALDH2 can reduce oxidative stress in hair follicle cells, which is one of the main causes of alopecia, thereby reducing the risk of alopecia and promoting hair growth.

In particular, the above results suggest that the activation of aldehyde dehydrogenase 2 can contribute not only to the growth of hair but also to the inhibition of gray hair occurrence. Specifically, gray hair is primarily caused by a decrease in pigment production due to aging of melanocytes and the loss of melanocytes due to the production of reactive oxygen species. The tyrosinase enzyme in melanocytes mediates the process of oxidizing tyrosine to DOPA in the melanin synthesis step, and this process occurs periodically in hair to produce melanin.

However, reactive oxygen species are generated during the melanin production process, and when the reactive oxygen species accumulate, the antioxidant system is damaged and oxidative stress is induced as cellular aging progresses. Melanocytes are vulnerable to the excessive accumulation of oxidative stress. In particular, it is known that accumulated oxidative stress may disrupt the homeostasis of melanocytes to induce the death or deformation of cells.

Therefore, in order to suppress the occurrence of gray hair, it is particularly important to reduce the exposure of melanocytes to reactive oxygen species, and the present inventors have confirmed through the above examples that the activation of aldehyde dehydrogenase 2 is a mechanism by which oxidative stress in human hair follicle cells is remarkably reduced. Therefore, upon treatment with an aldehyde dehydrogenase 2 activator, reactive oxygen species generated during the activation process of melanocytes may be reduced, thereby effectively inhibiting damage to melanocytes and the occurrence of gray hair due to aging.

### Example 6. Confirmation of changes in expression of ALDH2 and mitochondria according to anagen and telogen phases in human hair follicles

To confirm the changes in ALDH2 in hair follicles according to the telogen and anagen phases, immunofluorescence staining was performed on tissues from alopecia patients and healthy subjects. To identify aldehyde dehydrogenase 2 located in mitochondria, an antibody (anti-VDAC) targeting a mitochondrial outer membrane protein marker voltage-dependent anion channel (VDAC) and an aldehyde dehydrogenase 2 antibody (anti-ALDH2) were used. Each tissue was cut to a thickness of 4 µm, and then treated with anti-VDAC and anti-ALDH2 as primary antibodies, and treated with a fluorescently-labeled secondary antibody. Nuclei were stained using DAPI reagent, and then observed under a fluorescent microscope and photographed to confirm the number of VDAC- and ALDH2-positive cells.

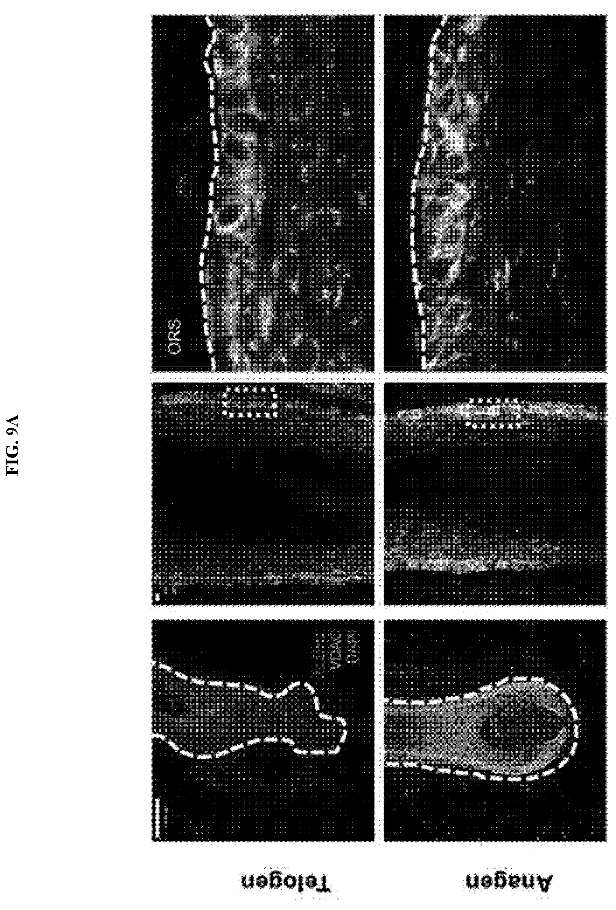

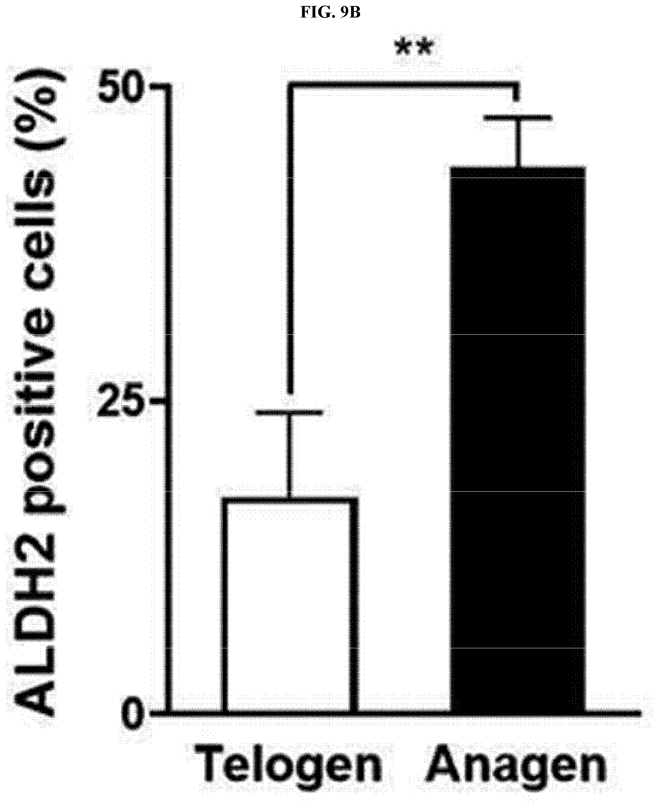

The results are shown in FIGS. 9A and 9B (in the fluorescent images, (VDAC, green fluorescence; ALDH2, red fluorescence; DAPI, blue fluorescence). While hair follicles in the telogen phase were observed in the tissue of alopecia patients, hair follicles in the anagen phase could be observed in the tissue of healthy subjects. In human hair follicles, the marker of the outer mitochondrial membrane and aldehyde dehydrogenase 2 were present at the same position. Further, it could be confirmed that aldehyde dehydrogenase 2 was expressed more in outer root sheath cells than in hair dermal papilla cells. Even when the proportion of aldehyde dehydrogenase 2-expressing cells located in the outer root sheath cells was re-confirmed, it could be similarly confirmed that the proportion was significantly increased in hair follicles during the anagen stage (FIG. 9B).

The above results suggest that the interaction between aldehyde dehydrogenase 2 and mitochondria in hair follicle cells is associated with the maintenance of the anagen phase of hair follicles and hair growth.

### Example 7. Confirmation of changes in mitochondrial activity in outer root sheath cells derived from human hair follicles

To confirm the changes in the mitochondrial activity according to the activity of aldehyde dehydrogenase 2 in outer root sheath cells derived from human hair follicles, the oxygen consumption rate was measured. After outer root sheath cells were plated in XF96 plates at 5×10³ cells per well, the control was treated with DMSO, and the experimental group was treated with Alda-1 (25 µM). After 12 hours, the concentration of oxygen dissolved in the medium during cell culture was measured using a real-time cell metabolism analyzer (Seahorse XFE96 Analyzer). Cells were washed twice with substrate oxidation assay media (XF DMEM pH 7.4 and 10 mM XF glucose, 1 mM XF pyruvic acid and 2 mM XF glutamine) and cultured at 37°C in the absence of CO₂ for 60 minutes. The oxygen consumption efficiency of the outer root sheath cells was measured after oligomycin (1.5 µM), FCCP (1.5 µM), and rotenone/antimycin A (final concentration of each = 0.5 µM) were sequentially injected into the XF96 plate port.

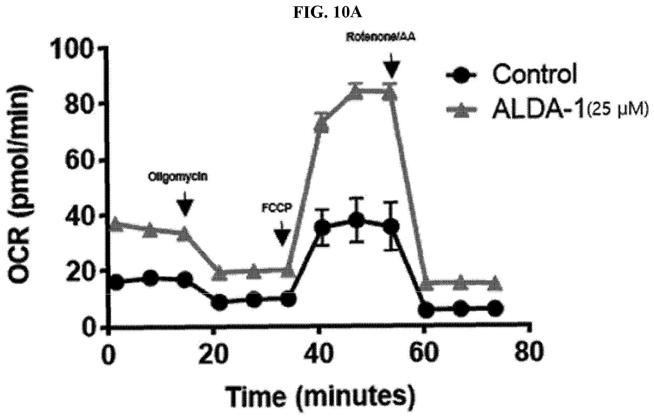

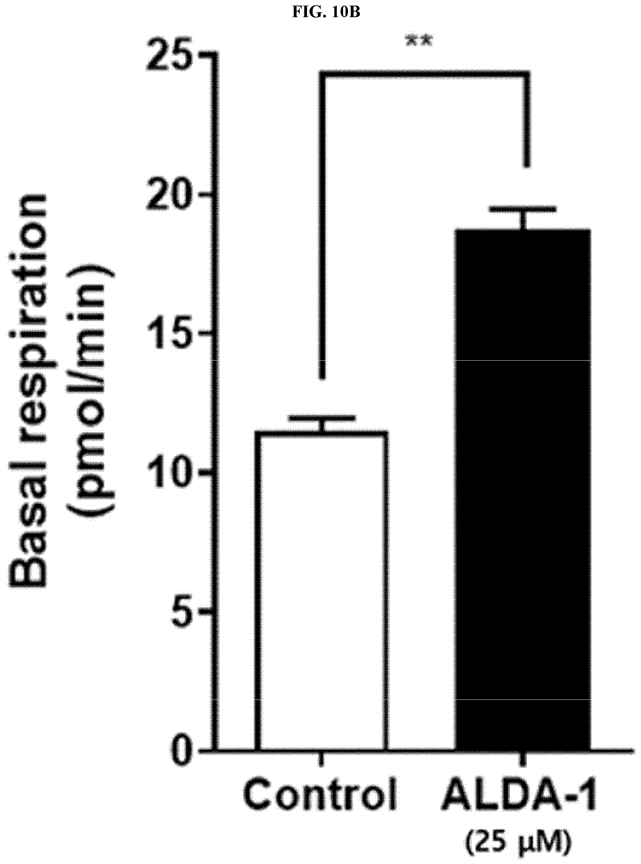

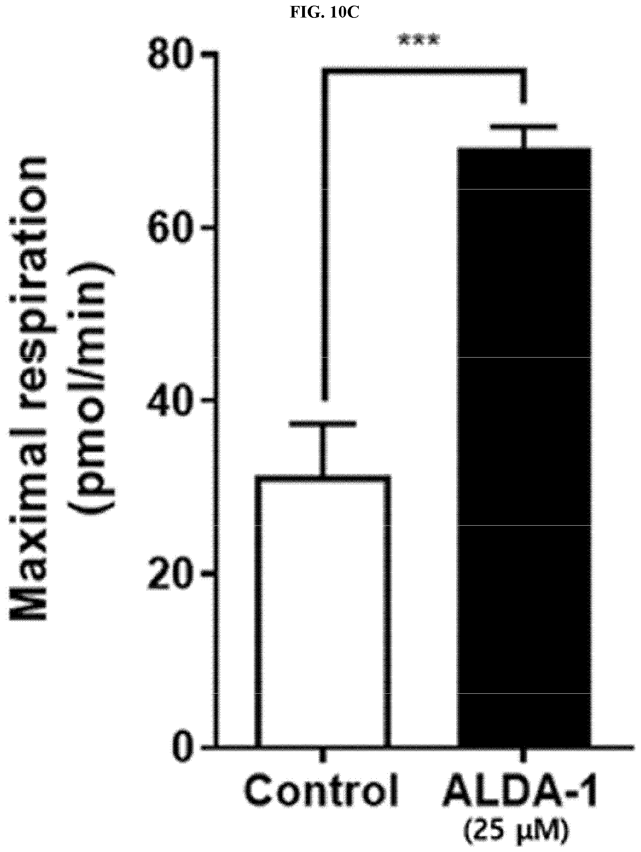

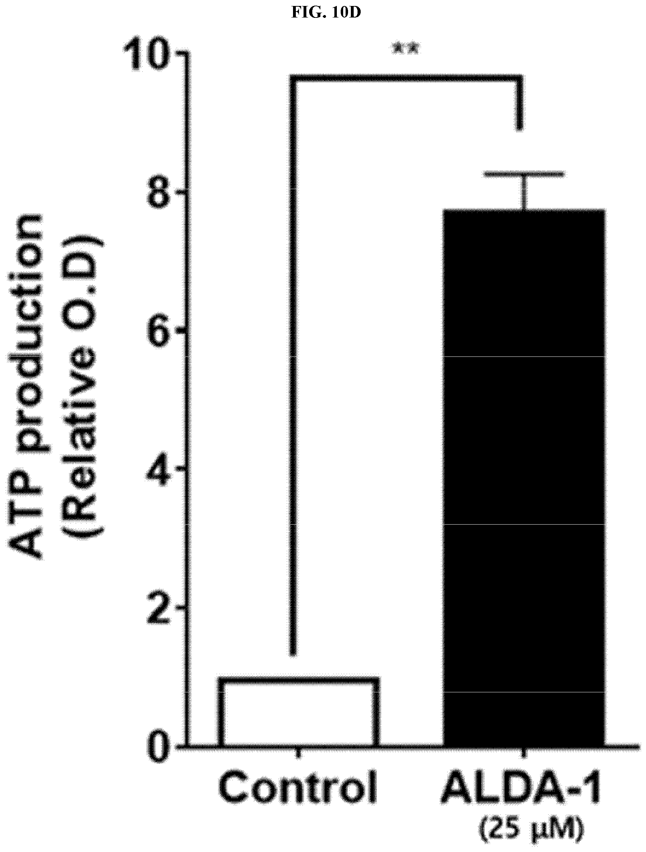

As a result, as shown in FIG. 10A, it could be confirmed that the oxygen consumption efficiency of outer root sheath cells increased as aldehyde dehydrogenase 2 was activated after treatment with Alda-1, and thus mitochondrial activity was increased. Further, the basal respiration rate of outer root sheath cells treated with Alda-1 was remarkably increased (FIG. 10B), and the maximal respiration rate was also remarkably increased (FIG. 10C). In addition, the ATP production of mitochondria increased nearly 10-fold after treatment with Alda-1 (FIG. 10D).

The above results support that the activation of aldehyde dehydrogenase 2 promotes hair growth by enhancing the function of mitochondria and inducing the anagen phase of hair follicles.

As described above, since it has been confirmed that the activation of ALDH2 may achieve excellent hair growth and hair regrowth promoting effects, it is expected that various ALDH2 activators will be utilized as agents for preventing, alleviating, and/or treating alopecia. In particular, since it has been confirmed that Alda-1 according to an embodiment of the present invention may effectively induce hair follicle and hair growth even at a much smaller dose than minoxidil which is an alopecia therapeutic agent in the related art, it is expected that the Alda-1 may be used as a safer and more economical alopecia therapeutic agent.

In addition, it was confirmed that the activation of ALDH2 and the mitochondrial interaction in hair follicles may contribute to the induction of the anagen phase of hair follicles and the promotion of hair growth, and in particular, it was confirmed that the activation of ALDH2 is a mechanism by which oxidative stress in hair follicles may be effectively inhibited. Therefore, the activation of ALDH2 not only may induce hair growth by reducing oxidative stress in hair follicles, but also may effectively inhibit the occurrence of gray hair.

### Example 8. Confirmation of combined effect of ALDH2 activator and minoxidil

Since it was confirmed that the ALDH2 activator may promote cell division in hair follicles and induce hair growth in previous examples, the alopecia therapeutic effect caused by the combined use of ALDH2 activator and minoxidil was confirmed in the present example.

Specifically, to confirm the hair growth effect caused by the combined use of the ALDH2 activator Alda-1 and minoxidil, it was confirmed whether the anagen phase of hair was induced after the two substances were administered in combination to a C57BL/6 mouse model. After the hair on the backs of eight-week-old C57BL/6 mice during the telogen phase was shaved, Alda-1 and/or minoxidil was (were) applied to the following groups: untreated control; Alda-1 (3 mM) single treatment group; 2% minoxidil single treatment group; and 3 mM Alda-1 and 2% minoxidil combined treatment group. A drug in which 3 mM Alda-1 and 2% minoxidil were mixed was applied to the combined treatment group. In the above mixed drug, Alda-1 and minoxidil were mixed at a weight ratio of 1 : 37. To both the single treatment groups and the combined treatment group, the drug was applied at a dose of 200 µl each time. Each drug or the mixture of drugs was applied to the backs of the mice once a day for three weeks, and the presence or absence of hair growth and the degree of induction of the anagen phase were confirmed by comparison with the control.

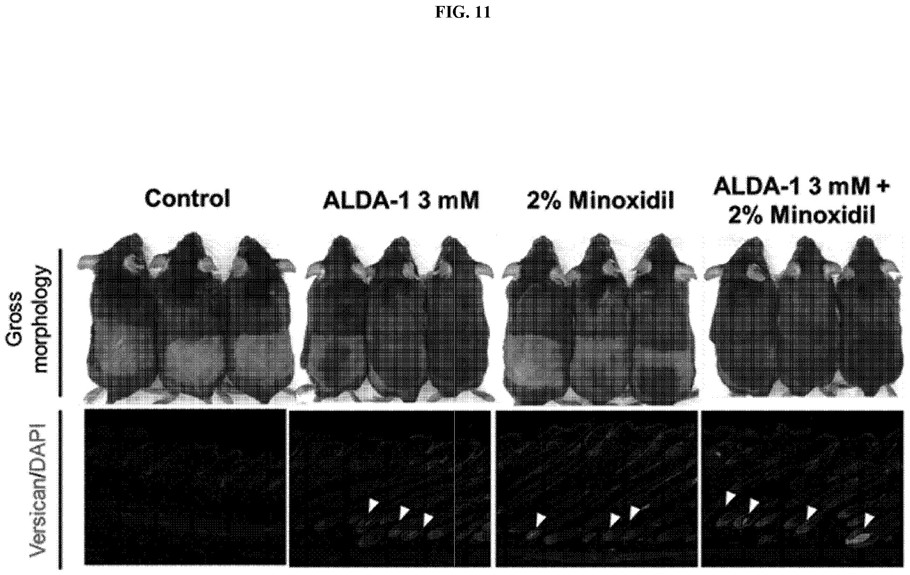

As a result, as shown in FIG. 11, hair growth was shown in all mice treated with each drug alone or in combination compared to the control, and in particular, the group treated with Alda-1 and minoxidil in combination showed a much larger area of hair growth in the anagen phase than the groups treated with each drug alone.

Further, skin tissue where hair grew during the anagen stage was fixed in paraffin and immunofluorescent staining was performed to confirm versican, which is a marker for hair dermal papilla cells involved in hair formation and growth, and as a result, compared to the groups treated with Alda-1 or minoxidil alone, a much stronger signal was detected in the group treated with Alda-1 and minoxidil in combination, confirming that the number of hair dermal papilla cells was remarkably increased .

The above results indicate that Alda-1 has excellent alopecia treatment effects by itself, and especially when Alda-1 is used in combination with minoxidil, a synergistic alopecia treatment effect may be exhibited by further enhancing the effects of promoting the induction of the hair anagen phase and the formation of hair follicles.

As described above, since it has been confirmed that the activation of ALDH2 may achieve excellent hair growth and hair regrowth promoting effects, it is expected that various ALDH2 activators will be utilized as agents for preventing, alleviating, and/or treating alopecia. In particular, since it has been confirmed that Alda-1 according to an embodiment of the present invention may effectively induce hair follicle and hair growth even at a much smaller dose than minoxidil which is an alopecia therapeutic agent in the related art, it is expected that the Alda-1 may be used as a safer and more economical alopecia therapeutic agent.

Furthermore, since it has been confirmed that when ALDH2 is used in combination with minoxidil, the formation of hair follicles and the growth of hair are increased more remarkably than when each drug is used alone, better alopecia treatment effects can be achieved using ALDH2 in combination with minoxidil. In particular, when ALDH2 of the present invention is used, the amount of minoxidil used, which has the risk of causing side effects, can be reduced, so it is expected that a safer and better alopecia treatment can be achieved.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described examples are illustrative only in all aspects and are not restrictive.

### [Industrial Applicability]

The present invention relates to a composition for preventing, alleviating and/or treating alopecia or gray hair (or poliosis), and the like, and was completed by confirming that an aldehyde dehydrogenase 2 (ALDH2) activator promotes hair follicle formation and hair growth and has an excellent effect on inhibiting the oxidative stress of hair follicles. Therefore, the ALDH2 activator according to the present invention can be used as a preparation for preventing, alleviating and/or treating alopecia and gray hair. Furthermore, combined treatment of the ALDH2 activator and minoxidil exhibited better alopecia prevention and treatment effects. Therefore, ALDH2 according to the present invention not only effectively promotes hair growth by itself, but also is expected to be usefully used in combination with minoxidil in the medical, beauty and food fields in order to prevent, alleviate and treat alopecia.

## Claims

1. A pharmaceutical composition for preventing or treating alopecia or gray hair, comprising an aldehyde dehydrogenase 2 (ALDH2) activator as an active ingredient.

2. The pharmaceutical composition of claim 1, wherein the composition is for promoting hair growth or hair regrowth.

3. The pharmaceutical composition of claim 1, wherein the alopecia is any one or more selected from the group consisting of alopecia areata, hereditary androgenetic alopecia, telogen effluvium, traumatic alopecia, trichotillomania, pressure alopecia, anagen alopecia, pityriasis alopecia, syphilitic alopecia, seborrheic alopecia, symptomatic alopecia, and congenital alopecia.

4. The pharmaceutical composition of claim 1, wherein the aldehyde dehydrogenase 2 activator is Alda-1 or a pharmaceutically acceptable salt thereof.

5. The pharmaceutical composition of claim 4, wherein the Alda-1 or the pharmaceutically acceptable salt thereof is contained at a concentration of 0.0001 to 50 mM with respect to the composition.

6. The pharmaceutical composition of claim 1, wherein the composition satisfies one or more characteristics selected from the group consisting of the following:
(a) promoting the proliferation of hair dermal papilla cells or outer root sheath cells;
(b) promoting the growth of hair follicles;
(c) inducing the anagen phase of hair; and
(d) increasing mitochondrial activity.

7. The pharmaceutical composition of claim 1, wherein the composition increases the level or activity of one or more proteins selected from the group consisting of bFGF, FGF-7, IGF-1, and PDGF.

8. The pharmaceutical composition of claim 1, wherein the compound reduces oxidative stress in hair dermal papilla cells or outer root sheath cells.

9. A cosmetic composition for preventing or alleviating alopecia or gray hair, comprising an aldehyde dehydrogenase 2 activator as an active ingredient.

10. The cosmetic composition of claim 9, wherein the composition is a formulation for external use in the form of a scalp treatment agent, soap, hair tonic, shampoo, rinse, hair pack, hair gel, lotion, conditioner, hair oil, mousse, cream, a solid agent, a solution, an emulsion, a dispersant, a micelle, a liposome, an ointment, toilet water, an essence, a patch, or a spray.

11. A quasi-drug composition for preventing or alleviating alopecia or gray hair, comprising an aldehyde dehydrogenase 2 activator as an active ingredient.

12. A food composition for preventing or alleviating alopecia or gray hair, comprising an aldehyde dehydrogenase 2 activator as an active ingredient.

13. A health functional food composition for preventing or alleviating alopecia or gray hair, comprising an aldehyde dehydrogenase 2 activator as an active ingredient.

14. A pharmaceutical composition for preventing or treating alopecia, comprising an aldehyde dehydrogenase 2 activator as an active ingredient, wherein the pharmaceutical composition is administered in combination with minoxidil, a pharmaceutically acceptable salt thereof, or an analog thereof.

15. The pharmaceutical composition of claim 14, wherein the pharmaceutical composition is administered simultaneously or sequentially with the minoxidil, the pharmaceutically acceptable salt thereof, or the analog thereof.

16. The pharmaceutical composition of claim 14, wherein the aldehyde dehydrogenase 2 activator enhances the alopecia treatment effect of the minoxidil, the pharmaceutically acceptable salt thereof, or the analog thereof.

17. The pharmaceutical composition of claim 14, wherein the aldehyde dehydrogenase 2 activator is Alda-1 or a pharmaceutically acceptable salt thereof.

18. A pharmaceutical composition for preventing or treating alopecia, comprising (a) an aldehyde dehydrogenase 2 activator; and (b) minoxidil, a pharmaceutically acceptable salt thereof, or an analog thereof, as active ingredients.

19. The pharmaceutical composition of claim 18, wherein the aldehyde dehydrogenase 2 activator is Alda-1 or a pharmaceutically acceptable salt thereof.

20. The pharmaceutical composition of claim 18, wherein the composition is in the form of a mixed preparation in which the (a) aldehyde dehydrogenase 2 activator; and the (b) minoxidil, a pharmaceutically acceptable salt thereof, or an analog thereof are mixed.

21. The pharmaceutical composition of claim 18, wherein the composition is in a form in which the (a) aldehyde dehydrogenase 2 activator; and the (b) minoxidil, a pharmaceutically acceptable salt thereof, or an analog thereof are each formulated and administered simultaneously, separately, or sequentially.

22. A cosmetic composition for preventing or alleviating alopecia, comprising (a) an aldehyde dehydrogenase 2 activator; and (b) minoxidil, a pharmaceutically acceptable salt thereof, or an analog thereof, as active ingredients.

23. The cosmetic composition of claim 22, wherein the composition is a formulation for external use in the form of a scalp treatment agent, soap, hair tonic, shampoo, rinse, hair pack, hair gel, lotion, conditioner, hair oil, mousse, cream, a solid agent, a solution, an emulsion, a dispersant, a micelle, a liposome, an ointment, toilet water, an essence, a patch, or a spray.

24. A quasi-drug composition for preventing or alleviating alopecia, comprising (a) an aldehyde dehydrogenase 2 activator; and (b) minoxidil, a pharmaceutically acceptable salt thereof, or an analog thereof, as active ingredients.

25. A food composition for preventing or alleviating alopecia, comprising (a) an aldehyde dehydrogenase 2 activator; and (b) minoxidil, a pharmaceutically acceptable salt thereof, or an analog thereof, as active ingredients.

26. A health functional food composition for preventing or alleviating alopecia, comprising (a) an aldehyde dehydrogenase 2 activator; and (b) minoxidil, a pharmaceutically acceptable salt thereof, or an analog thereof, as active ingredients.

27. A method for preventing, alleviating or treating alopecia or gray hair, the method comprising administering an aldehyde dehydrogenase 2 activator to a subject in need thereof.

28. A use of an aldehyde dehydrogenase 2 activator for preventing, alleviating, or treating alopecia or gray hair.

29. A use of an aldehyde dehydrogenase 2 activator for preparing a drug for preventing, alleviating, or treating alopecia or gray hair.

30. A method for preventing or treating alopecia, the method comprising administering, to a subject in need thereof, (a) an aldehyde dehydrogenase 2 activator; and (b) minoxidil, a pharmaceutically acceptable salt thereof, or an analog thereof.

31. A use of a composition comprising (a) an aldehyde dehydrogenase 2 activator; and (b) minoxidil, a pharmaceutically acceptable salt thereof, or an analog thereof, for preventing, alleviating, or treating alopecia.

32. A use of a composition comprising (a) an aldehyde dehydrogenase 2 activator; and (b) minoxidil, a pharmaceutically acceptable salt thereof, or an analog thereof, for preparing a drug for preventing, alleviating, or treating alopecia.
